# EUROPEAN PATENT APPLICATION

(11) **EP 3 670 652 A1**
(43) Date of publication of application: **24.06.2020**
(21) Application number: 19217097.5
(22) Date of filing: 17.12.2019
(51) Int. Cl.: C12N 7/04

(54) **VLP FOR THE TREATMENT OF A LYSOSOMAL STORAGE DISEASE**

(30) Priority: 18.12.2018 WO PCT/EP2018/085699; 26.04.2019 WO PCT/EP2019/060814
(71) Applicant: NEUWAY Pharma GmbH, 53175 Bonn (DE)
(72) Inventor: Demina, Victoria, 53175 Bonn (DE); Stapf, Marcus, 53175 Bonn (DE); Sotnikov, Sergey, 81735 München (DE); Manninga, Heiko, 37073 Göttingen (DE)
(74) Representative: Hemsath, Lars

(57) **Abstract**

The invention relates to virus like particles (VLP) associated with a lysosomal enzyme or an expression vector encoding a lysosomal enzyme which are used in a method for the treatment of a lysosomal storage disease. The invention also relates to a pharmaceutical composition for use in a method for the treatment of a lysosomal storage disease, to an expression vector encoding a lysosomal enzyme and to a method of associating a VLP with an expression vector encoding a lysosomal enzyme.

## Description

### FIELD OF THE INVENTION

The invention relates to virus like particles (VLP) associated with a lysosomal enzyme or an expression vector encoding a lysosomal enzyme which are used in a method for the treatment of a lysosomal storage disease in a subject in the need thereof, preferably a human. The invention also relates to a pharmaceutical composition for use in a method for the treatment of a lysosomal storage disease, to an expression vector encoding a lysosomal enzyme and to a method of associating a VLP with an expression vector encoding a lysosomal enzyme.

### BACKGROUND OF THE INVENTION

Lysosomal storage diseases (LSD) are a group of about 50 inherited diseases. LSD mostly involve the dysfunction of lysosomal hydrolases, which result in impaired substrate degradation. As a consequence, incompletely processed cellular material accumulates within various tissue types, and disease-specific clinical manifestations occur.

Beyond substrate degradation, lysosomes are involved in energy homeostasis, generation of building blocks for cell growth, mitogenic signaling, the priming of tissues for angiogenesis and metastasis formation and activation of transcriptional programs.

One example of a lysosomal storage disease is the metachromatic leukodystrophy (MLD). MLD is characterized by a diminished activity of arylsulfatase A (ASA) caused by mutations in the ASA gene affecting the metabolism of sphingolipids. Without ASA, sulfatides build up in many tissues of the body, eventually destroying the myelin sheath of the nervous system. Symptoms involve developmental delays, weakness, muscle rigidity, mental deterioration, dementia and blindness.

There are three clinical subtypes of MLD, based on the age of the subject (human) at the onset of the first symptoms (van Rappard et al., Best Pract Res Clin Endocrinol Metab. 2015;29(2):261-73):
The "late-infantile form" has its onset typically before 30 months of age of the child. It is characterized by rapid accumulation of sulfatides and progression of psychomotor regression. Death typically occurs within a few years after the onset of symptoms. No functional enzyme is present.

In the "juvenile variant" symptoms start between 2.5 and 16 years of age. In the beginning, disease progression is slower than in the late-infantile form, but once neurological signs become more evident, the decline is rapid and patients eventually lose all skills. The end stage of the disease can last several years, and its duration is variable. Most patients carry one ASA allele that allows for expression of low amounts of functional enzyme.

The "adult variant" has an insidious onset after the age of 16 years. Disease progression is generally slower than in the infantile and juvenile form. Death occurs within decades after disease onset. Patients carry two mutations, allowing for the expression of low amounts of functional enzyme, which delays the process of sulfatide accumulation and thereby the onset of the disease.

The following gene therapeutic approaches have been discussed in the prior art for the treatment of MLD:
- bone marrow transplantation of genetically modified autologous hematopoietic stem cells involving re-administration of a patient's bone marrow stem cells genetically modified using lentiviral vectors for stable expression of ASA
- cell therapy involving delivery of microencapsulated recombinant cells that overexpress the ASA gene
- in vivo gene therapy, namely the delivery of an optimised adenoviral (or adeno-associated) or lentiviral vector encoding hASA directly to the CNS to provide long-term, sustained and persistent correction of the genetic defect (Rosenberg at al., J Neurosci Res. 2016;94(11):1169-79).

The following non-gene therapeutic approaches have been discussed in the prior art for the treatment of MLD:
- enzyme replacement therapy involving intravenous, intracerebroventricular or intrathecal injection of the ASA enzyme
- hematopoietic stem cell therapy involving donor hematopoietic stem cells crossing the blood brain barrier (BBB) and differentiating into microglial cells to produce wildtype ASA
- small molecule-based therapies, for example, application of N-butyldeoxynojirimycin (NB-DNJ) miglustat (Zavesca®, Actelion Pharmaceuticals Ltd, Allschwil, Switzerland) or Warfarin (coumadin)
- substrate reduction therapy.

These treatment attempts are rather unspecific, meaning that the drugs do not specifically target the CNS, but are equally distributed over the patient's body after administration. In addition, safety concerns arise when, for example, viral vectors are administered.

Hence, there exists a need for safe and effective therapies for the treatment of lysosomal storage diseases. It is thus an objective of the present invention to provide means for a novel therapy for the treatment of lysosomal storage diseases, in particular of MLD, which overcomes at least one of these constraints.

### SUMMARY OF THE INVENTION

The inventors have found that virus-like particles (VLP), more specifically VLP of the JC virus, associated with a lysosomal enzyme or an expression vector encoding a lysosomal enzyme, preferably incorporating a lysosomal enzyme or incorporating an expression vector encoding a lysosomal enzyme, are particularly well suited for the treatment of lysosomal storage diseases, such as MLD. The VLP according to the invention can be used for the efficacious delivery of functional lysosomal enzymes or plasmids encoding such enzymes into the CNS in a patient in the need thereof. To achieve this, according to the invention, the VLP is associated with a lysosomal enzyme or an expression vector encoding a lysosomal enzyme, whose absence or reduced activity is responsible for the LSD to be treated.

The VLP according to the invention can effectively cross the blood brain barrier (BBB), advantageously even the physiologically intact BBB. Hence the VLP associated with a lysosomal enzyme or an expression vector encoding a lysosomal enzyme can effectively deliver the lysosomal enzyme or the expression vector encoding the lysosomal enzyme into the CNS.

The inventors found ASA mRNA in the brain of mice after intravenous administration of the VLP according to the invention. They also found ASA protein in the brain thereafter. Thereby, it can be concluded that with the administration of VLP according to the invention the lysosomal enzyme activity in the CNS can be enhanced. The enhanced lysosomal enzyme activity enables the effective treatment of the lysosomal storage diseases; if the lysosomal enzyme is ASA the treatment of MLD becomes possible.

Surprisingly, it has been found that the VLP according to the invention target specifically the CNS, i.e. they do not equally distribute over the body after having been administered to the patient, e.g. intravenously. That means that a larger proportion of VLP according to the invention is found in the CNS than outside the CNS.

The VLP according to the invention particularly target astrocytes, oligodendrocytes, neurons and/or microglia. A specifically preferred target of the VLP of the invention are oligodendrocytes.

The VLP according to the invention are also stable and homogeneous which is especially important for clinical use because it allows for a better quality management and standardization of the drug product.

Thus, it has been shown by the inventors, that VLP, in particular VLP of the JC virus, associated with a lysosomal enzyme or an expression vector encoding a lysosomal enzyme can be used to provide an efficacious and safe treatment of LSD, in particular of MLD.

It has also been found that VLP derived from JCV with an improved efficacy can be provided, when the method for the production of said VLP comprises twice the steps of disassembling the VLP into pentamers and reassembling the pentamers into a VLP.

The method of incorporating a lysosomal enzyme, preferably human arylsulfatase A, or incorporating an expression vector encoding a lysosomal enzyme, preferably human arylsulfatase A, into a VLP preferably comprises the following steps:
a) providing a composition comprising VP1 proteins,
b) exposing the VP1 proteins of the composition of a) to conditions inducing the VP1 to assemble into VLP,
c) exposing the VLP of the composition of b) to conditions disassembling the VLP into pentamers,
d) exposing the pentamers of the composition of c) to conditions inducing the pentamers to reassemble into VLP
e) exposing the VLP of the composition of d) to conditions disassembling the VLP into pentamers,
f) exposing the pentamers of the composition of e) to the lysosomal enzyme or the expression vector to conditions inducing the pentamers to assemble into a VLP associated with the lysosomal enzyme or the expression vector.

An improved efficacy in particular means that more cargo, i. e. in particular lysosomal enzyme, preferably human arylsulfatase A, or an expression vector encoding a lysosomal enzyme, preferably human arylsulfatase A, reaches the CNS. The improved efficacy can have manifold reasons, which might interact. The higher efficacy can for example be due to a more efficacious crossing of the BBB by the VLP, so that a higher amount of VLP and/or cargo enters the CNS. It can equally be due an improved release of the cargo from the VLP after reaching the CNS. Furthermore, a reason for the higher efficacy can be the fact that each individual VLP can be loaded with a higher amount of cargo or the overall amount of loaded VLP can be increased.

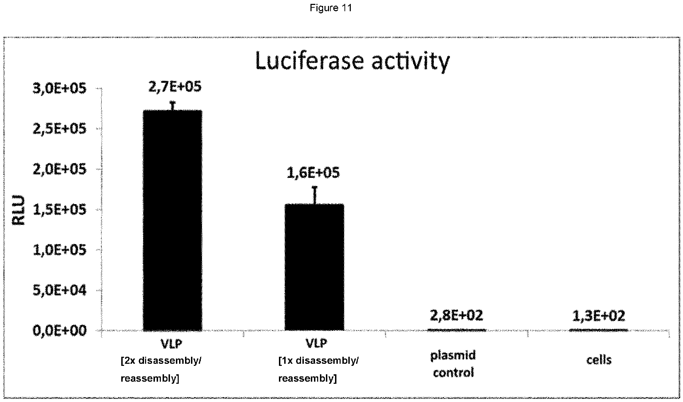

The improved efficacy of the VLP obtainable by the method according to the invention or a drug delivery system obtainable by the method according to the invention is exemplarily demonstrated by the increased expression of luciferase, when the cargo of the VLP is the luciferase expression plasmid NanoLuc® (Example 11 below, Fig. 11).

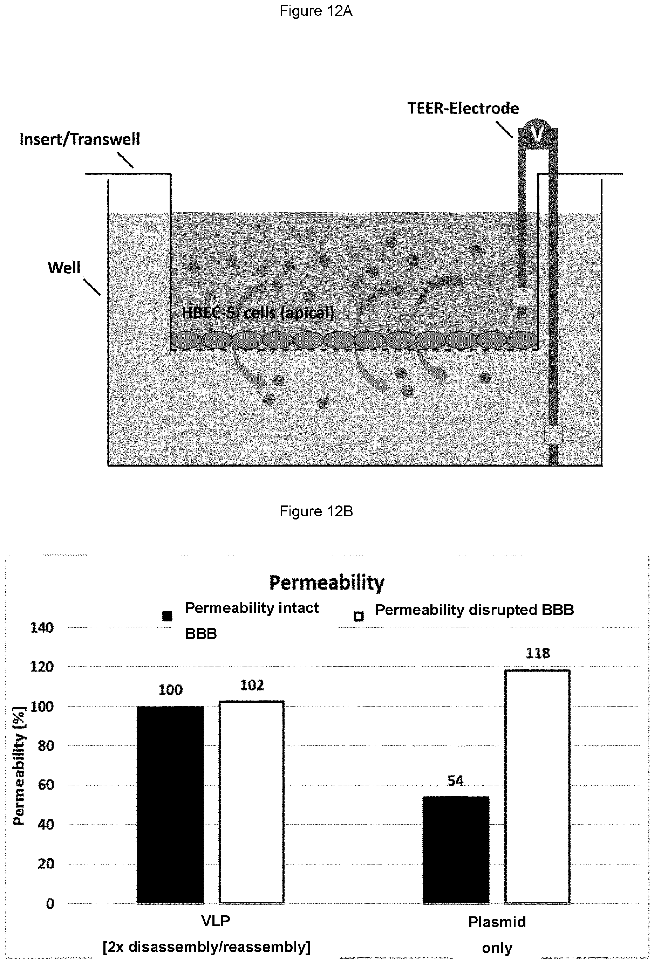

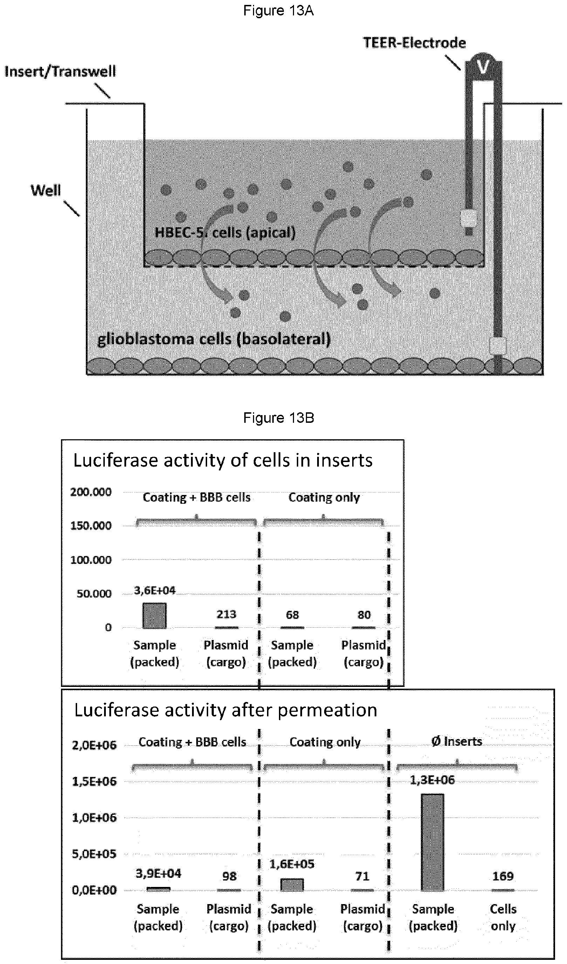

Example 11 furthermore gives evidence that the VLP obtained in step f) efficiently cross the BBB (Example 11 below, Figs. 12 and 13). Therewith, evidence is provided, that the VLP can be used as a drug delivery system for the transport of a drug into the CNS.

Moreover, it has been found, that the VLP obtained in step d), are particularly suitable for storage. They withhold storage conditions of -80 °C for a period of more than 24 h. After storage the VLP can be further processed, for example for providing the drug delivery system. Hence they represent a suitable storable intermediate product during the manufacture of a drug delivery system comprising VLP associated with a cargo.

In one particular aspect, thus, the invention relates to a method for providing VLP with an increased suitability for storage. Such VLP, for practical reasons, substantially facilitate the production of the drug delivery system. It allows for the large-scale manufacture of VLP and, then, their intermediate storage without cargo. After storage, on demand of the individual costumer, the stored VLP can be further processed and loaded with the desired cargo. Therefore, the intermediate storage of the VLP provided in step d) enables more flexibility in the manufacturing process of a drug delivery system on the basis of VLP.

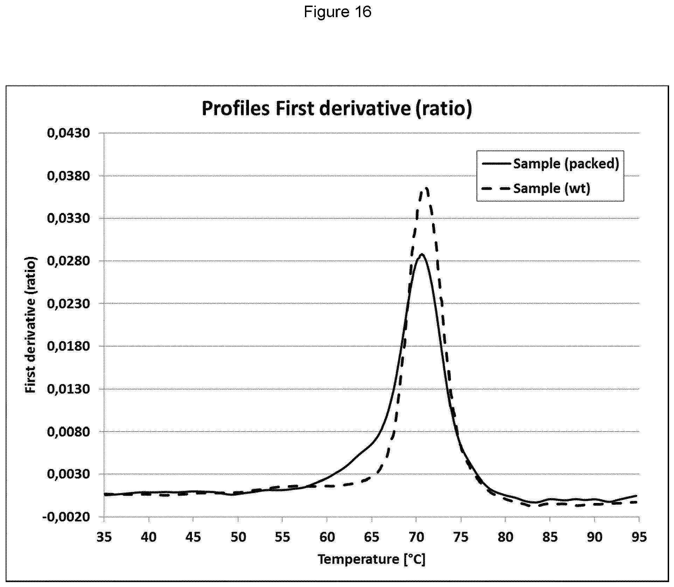

Furthermore, it has been found that the VLP according to the invention are stable. Their stability can be equally compared with the VLP which are the result from VP1 production and a direct subsequent assembly, i.e. without a disassembly/reassembly step (Fig. 16). Therefrom it follows, that the two disassembly and reassembly steps according to the invention, surprisingly, do not have a negative impact on the stability of the VLP.

In yet another aspect, the invention relates to a drug delivery system or a composition comprising VLP.

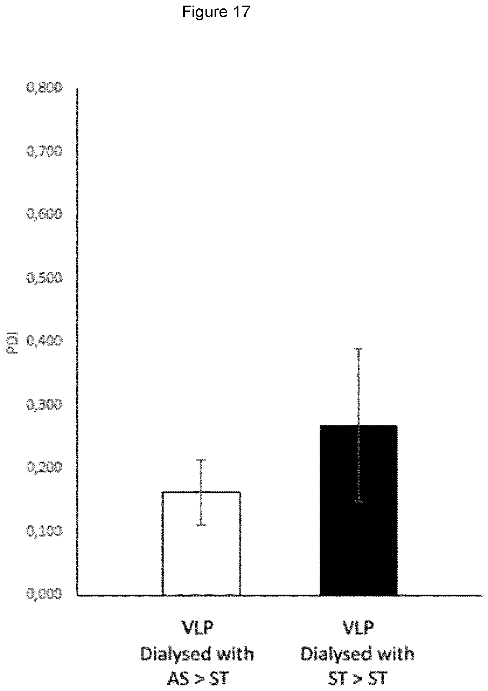

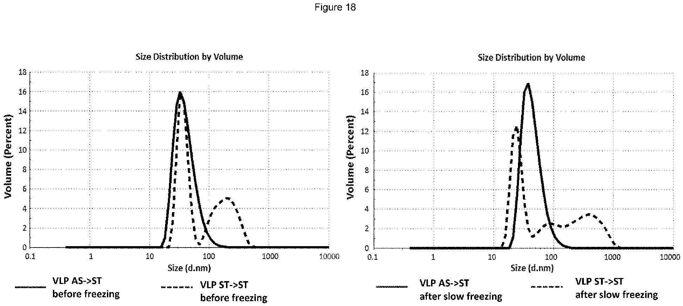

It was further surprisingly found, that the homogeneity of the composition comprising VLP can be improved by reassembling the pentamers of step d) in two steps, whereas the first step comprises inducing the aggregation of the pentamers of step c) and the second step comprises separating the pentamers from the conditions inducing the aggregation of the pentamers (Example 13 below, Figs. 17 and 18).

A homogeneous size distribution of the composition comprising VLP is advantageous because it allows producing a defined population of VLP for use as the drug delivery system which is important to fulfill a defined quality standard.

### BRIEF DESCRIPTION OF THE FIGURES

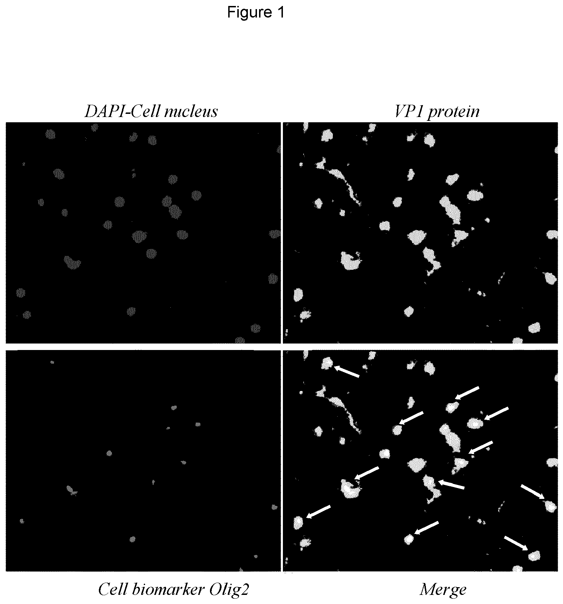

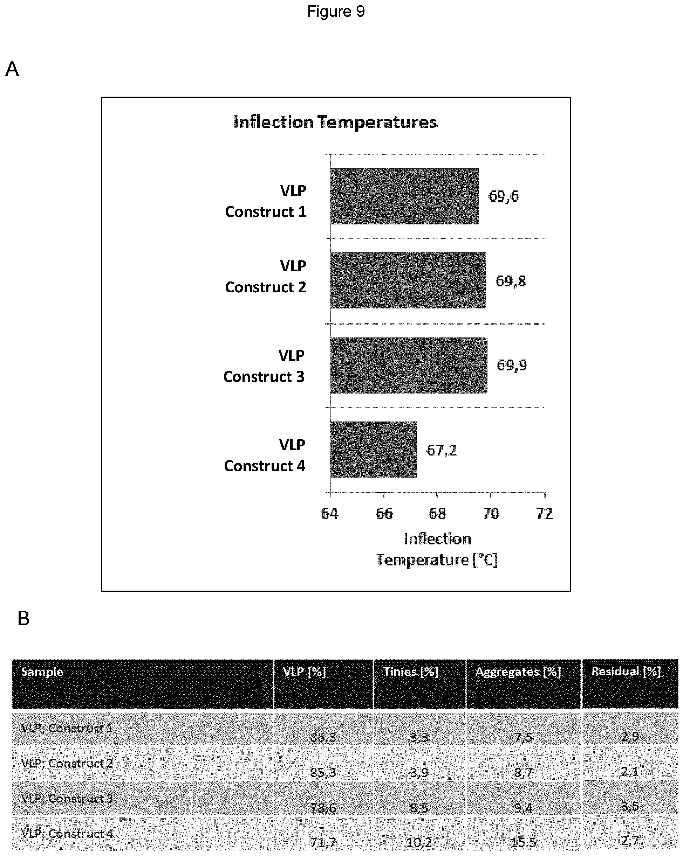

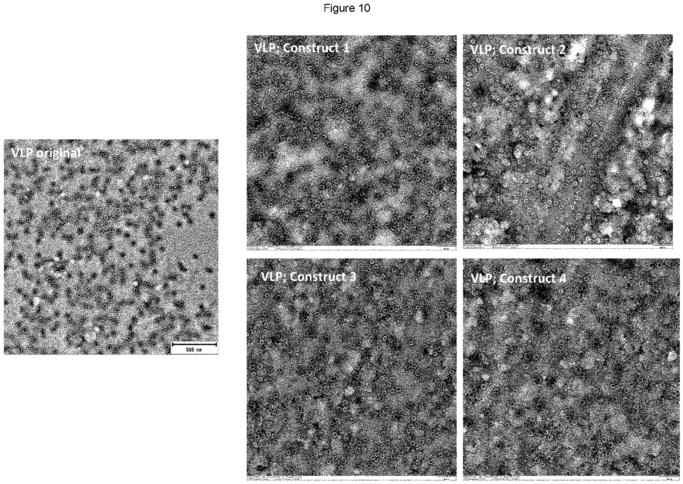

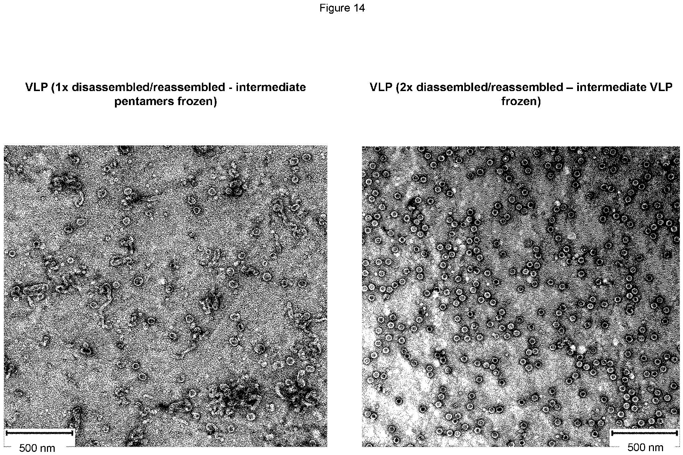

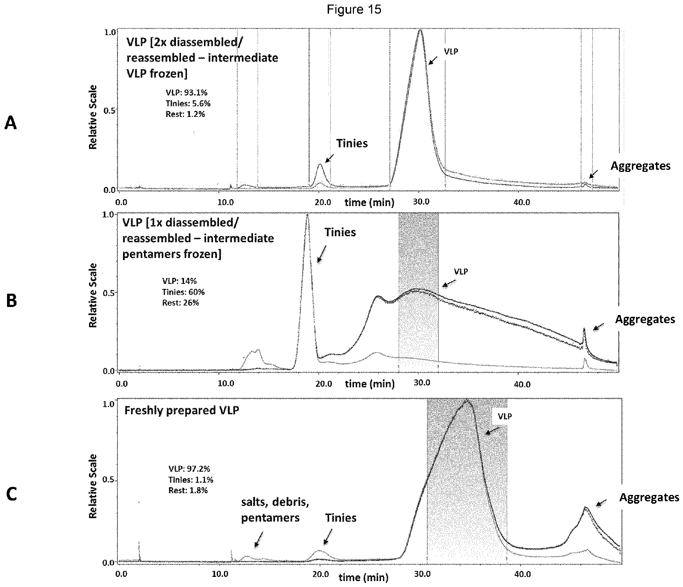

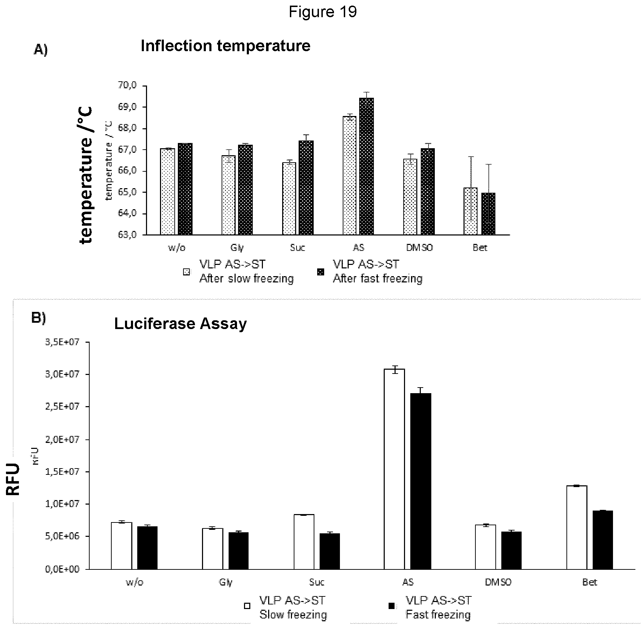

**Fig. 1** **Co-localization of VP1 protein with the oligodendrocyte marker Olig2.**
   Upper left panel shows staining of the cell nuclei with the dye DAPI. Upper right panel shows the localization of the VP1 protein (FITC-fluorescence). Lower left panel shows the staining with the anti-Olig2 antibody (TRITC-fluorescence). Lower right panel represents the merged pictures of the staining for the Olig2-marker and the VP1 protein. Cells which are positive for VP1 and Olig2 are marked with an arrow.

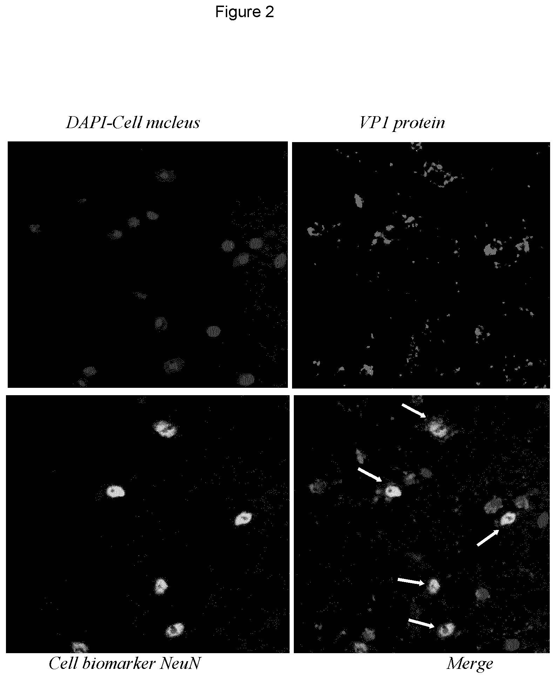

**Fig. 2** **Co-localization of VP1 protein with the neuronal marker NeuN.**
   Upper left panel shows staining of the cell nuclei with the dye DAPI. Upper right panel shows the localization of the VP1 protein (FITC-fluorescence). Lower left panel shows the staining with the anti-NeuN antibody (TRITC-fluorescence). Lower right panel represents the merged pictures of the staining for the NeuN-marker and the VP1 protein. Cells which are positive for VP1 and NeuN are marked with an arrow.

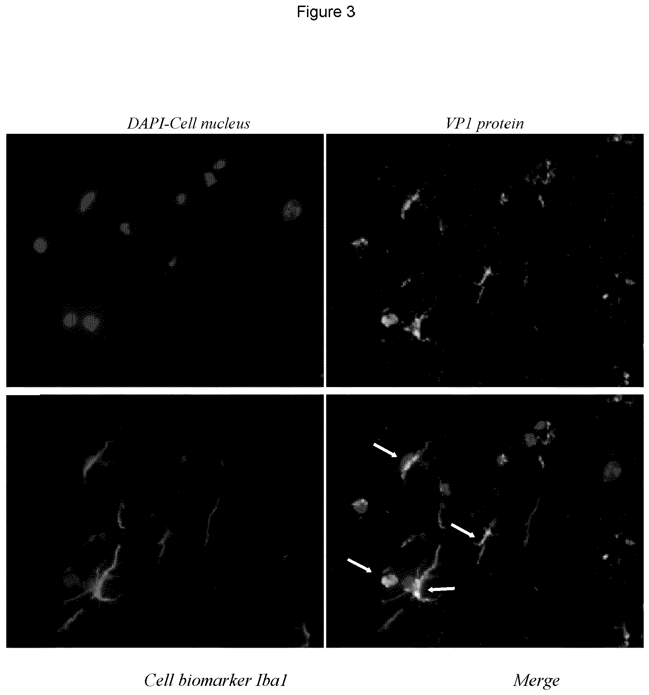

**Fig. 3** **Co-localization of VP1 protein with the microglia marker Iba1.**
   Upper left panel shows staining of the cell nuclei with the dye DAPI. Upper right panel shows the localization of the VP1 protein (FITC-fluorescence). Lower left panel shows the staining with the anti-Iba1 antibody (TRITC-fluorescence). Lower right panel represents the merged pictures of the staining for the Iba1-marker and the VP1 protein. Cells which are positive for VP1 and Iba1 are marked with an arrow.

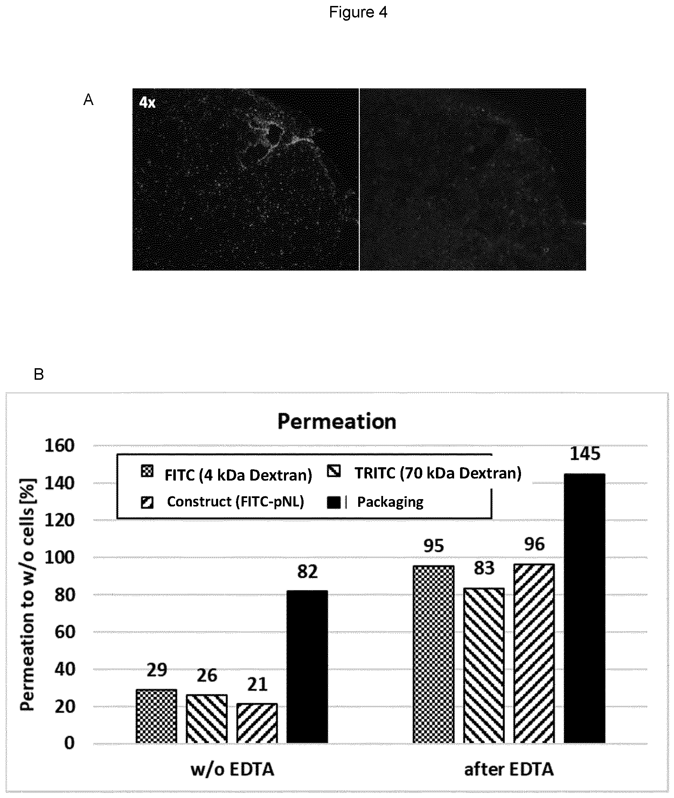

**Fig. 4** **Permeation of VLP associated with a pNL plasmid in an *in vitro* BBB model**
   A: DAPI (left) and WGA (right) staining of BBB cells in an *in vitro* BBB model.
   B: Permeation of fluorescence dyes (FITC, TRITC), a fluorescent dye coupled to a plasmid (FITC-pNL) and a VLP associated with a pNL plasmid. Permeation in % in relation to a permeation without cells. Left four columns without EDTA, right four columns with EDTA.
**Fig. 5** **ASA mRNA analysis in liver and brain of healthy mice injected with VLP associated with an expression vector encoding ASA**
   Analysis of ASA mRNA levels in liver and brain of healthy mice at 72 and 120 h after injection of VLP associated with an expression vector encoding ASA. Fold increase compared with vehicle (buffer). Numbers in parentheses denote the respective number of mice.
**Fig. 6** **Single mouse analysis (healthy mice) - ASA mRNA detection in the brain** Single mouse analyses of ASA mRNA after 72 h (top) and 120 h (bottom) after injection of VLP associated with an expression vector encoding ASA. Numbers denote single mice. Relative fold regulation means gene upregulation (expression) in comparison to vehicle control group.
**Fig. 7** **Single mouse analysis (healthy mice) - Western Blot analysis (hASA detection in the brain) and corresponding qPCR**
   Single mouse analysis of ASA protein (Western blot, top) in the brain after injection of VLP associated with an expression vector encoding ASA and corresponding qPCR analysis (bottom). Vehicle (buffer), plasmid and plasmid + VLP are compared. Numbers denote single mice. Relative fold regulation means gene upregulation (expression) in comparison to vehicle control group.

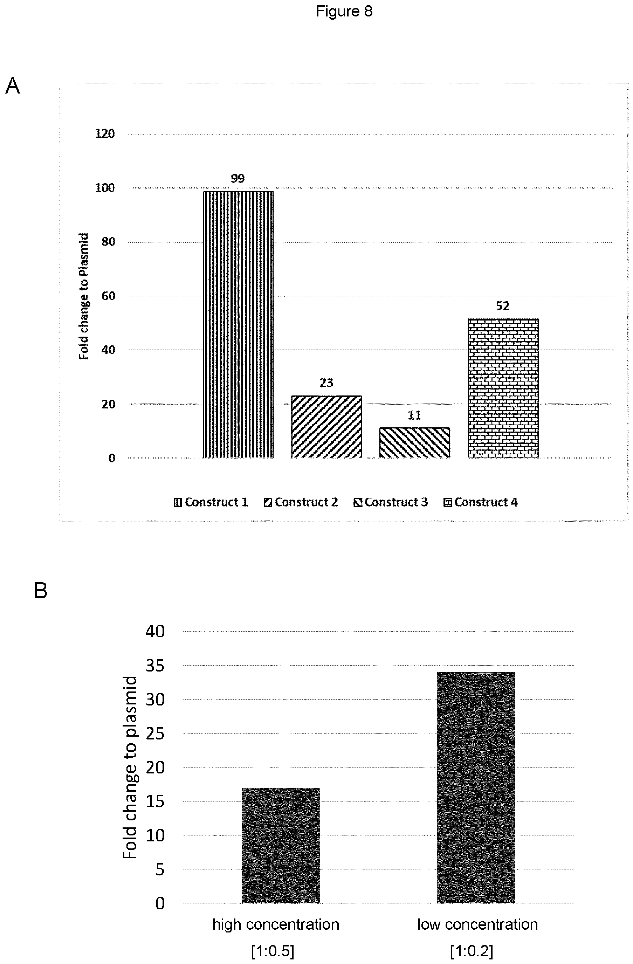

**Fig. 8** **Expression of human ASA in glioblastoma cells by qPCR.**
   qPCR analyses of human ASA expression after incubation of glioblastoma cells with VLP associated with hASA expression vectors.
   A: VLP associated with four different hASA expression vectors at a packaging ratio of VLP: plasmid of 1:0.2
   B: VLP associated with Construct 4 of A at two different packaging ratios (high concentration [VLP: plasmid of 1:0.5] and low concentration [VLP: plasmid of 1:0.2]
**Fig. 9****. nDSF and AF4 analyses of VLP associated with four different hASA expression vectors**
   A: nDSF analyses of four samples (VLP associated with the expression vectors Constructs 1-4)
   B: AF4 analyses of the same four samples
**Fig. 10****. TEM images of VLP associated with four different hASA expression vectors**
   Comparison of VLP as such ("VLP original": not associated with a cargo) (left) and VLP associated with four different expression vectors (Constructs 1-4, right).
**Fig. 11** **Luciferase activity comparing differently prepared VLP**
   Luciferase activity measured in glioblastoma cells transfected either with VLP prepared with two rounds of disassembly and reassembly or with VLP having been disassembled and reassembled only once
**Fig. 12** **BBB model as a monoculture**
   Setup (A) of a BBB model as a monoculture and permeability (B) of VLP prepared with two rounds of disassembly and reassembly
**Fig. 13** **BBB model as a coculture**
   Setup (A) of a BBB model as a coculture and permeability (B) of VLP prepared with two rounds of disassembly and reassembly
**Fig. 14** **TEM images of VLP**
   TEM images of VLP prepared with two rounds of disassembly/reassembly *versus* VLP prepared with one round of disassembly and reassembly, and with intermediate storage
**Fig. 15** **FFF-MALS analyses of VLP**
   FFF-MALS analyses of VLP prepared with two rounds of disassembly and reassembly with intermediate storage of the VLP (A), VLP with one round of disassembly and reassembly with intermediate storage of the pentamers (B) and freshly prepared VLP (C).
**Fig. 16** **nDSF analyses of VLP**
   nDSF analyses of freshly prepared VLP and VLP prepared with two rounds of disassembly and reassembly with intermediate storage
**Fig. 17** **DLS analyses showing the PDI of VLP**
   DLS analyses showing the PDI of VLP according to step d) of the method comprising two rounds of disassembly and reassembly with or without inducing aggregation of pentamers before storage
**Fig. 18** **DLS analyses showing the average diameter of VLP**
   DLS analyses showing the average diameter of VLP according to step d) of the method comprising two rounds of disassembly and reassembly with or without inducing aggregation of pentamers before storage and after storage
**Fig. 19** **nDSF analyses and Luciferase activity of VLP stored with different cryoadditives**
   nDSF analyses (A) and Luciferase activity (B)

### DETAILED DESCRIPTION OF THE INVENTION

In a first aspect of the invention, the invention relates to VLP associated with a lysosomal enzyme or an expression vector encoding a lysosomal enzyme for use in a method for the treatment of a lysosomal storage disease in a subject, in particular a human. Hence the invention also relates to a method of treating a human suffering from LSD with a VLP associated with a lysosomal enzyme or an expression vector encoding a lysosomal enzyme. The lysosomal enzyme is preferably ASA. The LSD is preferably MLD.

VLP as such, i. e. not associated with a cargo, do not comprise any genetic material because they are only built up of proteins and are otherwise "empty". VLP according to the present invention are associated with a lysosomal enzyme or an expression vector encoding a lysosomal enzyme. In a preferred embodiment, the VLP according to the present invention do not comprise viral genetic material encoding a viral protein. In this embodiment, the VLP may comprise a viral regulatory element as viral genetic material.

Viral genetic material comprises viral genetic material encoding a viral protein and viral regulatory elements as viral genetic material. Viral genetic material is derived from the nucleic acid of a virus, i. e. at least 70 % identical to a viral RNA or DNA. Preferably, the VLP according to the invention do not comprise any viral genetic material.

Thus, preferably, in case the VLP according to the invention comprise RNA or DNA, the RNA or DNA is not derived from a virus, i. e. the RNA or DNA is not viral genetic material, in particular the RNA or DNA does not encode a viral protein. It is particularly preferred that the RNA or DNA does not encode a viral protein and does not comprise a viral regulatory element.

In a preferred embodiment, the VLP according to the invention is associated only with an expression vector which only encodes a lysosomal enzyme, i. e. the expression vector does not encode any other protein. In a preferred embodiment, the expression vector does not encode a viral protein. It is particularly preferred that the expression vector does not encode a viral protein and does not comprise a viral regulatory element.

Preferably, the subject is an animal or a human being, preferably a human being.

The VLP according to the invention, advantageously, enable the delivery of the lysosomal enzyme or the expression vector encoding a lysosomal enzyme to a site of interest ("target"), preferably in a human. Preferably, the delivery is selective for the target, i.e. a higher proportion of the lysosomal enzyme or the expression vector encoding a lysosomal enzyme is delivered to the target than to other sites of the body or organ.

The target is preferably the CNS. CNS refers to the spinal cord and the brain, in particular to the brain. The term "brain" includes anatomical parts thereof, such as frontal lobe, parietal lobe, temporal lobe, occipital lobe, and cerebellum.

It is particularly advantageous if the lysosomal enzyme or the expression vector encoding a lysosomal enzyme is delivered to and/or into a target cell, preferably a target cell in the CNS, in particular an astrocyte, an oligodendrocyte, a neuron and/or microglia. In a particularly preferred embodiment, the target cell is an oligodendrocyte. Hence, the lysosomal enzyme or the expression vector preferably enters astrocytes, oligodendrocytes, microglia or neurons, in particular oligodendrocytes.

It is particularly advantageous when the target cell is contacted with an effective amount of the lysosomal enzyme. An effective amount of the lysosomal enzyme means that the amount is sufficient to enhance the activity of the enzyme whose absence causes the LSD.

Enzyme activity in the subject is usually measured in *in vitro* probes, typically in probes derived from solid tissues, leukocytes, fibroblasts, cultured amniotic fluid cells, serum, amniotic fluid, urine or tear drops to which a substrate is added. For example, for arylsulfatase A typical substrates include *p*-nitrocatechol sulfate, sulfatide, in particular [3H]-labelled sulfatide, lactosylceramide sulfate, galactosyl-sphingosine sulfate, ascorbic acid 2-sulfate or seminolipid (Raghavan et al., J Neurochem. 1981;36(2):724-31). In particular, *p*-nitrocatechol sulfate can be used as a substrate to measure arylsulfatase A and B activity in the urine of a subject (Baum et al., Clin Chim Acta. 1959;4(3):453-5).

In a particularly preferred embodiment, the VLP according to the invention cross the blood-brain barrier, preferably the physiologically intact blood-brain barrier, to enter the CNS together with the lysosomal enzyme or the expression vector. In other words, the VLP according to the invention preferably cross the BBB without a prior increase of the permeability of the BBB. The VLP according to the invention are capable of crossing the physiologically intact BBB.

The crossing of the BBB is especially advantageous if the target is the CNS, in particular if the target cell is an astrocyte, an oligodendrocyte, neuron and/or microglia. Hence, the VLP according to the invention can be used in a method of treatment of a lysosomal storage disease, wherein the method does not comprise a prior step of increasing the permeability of the BBB of the subject to be treated. The VLP according to the invention, preferably, is administered to a patient who has not received before administration any chemical or physical treatment for impairing or disrupting the BBB.

In a further embodiment, thus, the VLP associated with a lysosomal enzyme or an expression vector encoding a lysosomal enzyme or the pharmaceutical composition comprising the VLP according to the invention are free of any additive that can impact the permeability of the BBB.

The integrity of the BBB *in vitro* may be measured by known methods, for example by relative transendothelial electrical resistance measurement (TEER) (Rempe et al., Biochem Bioph Res Comm 2011, 406 (1): 64-69). Many *in vitro* models of BBB are established, including primary bovine or human brain endothelial cells in different cocultures, for example the human brain endothelial cell line HBEC-5i. *In vivo,* imaging methods, such as CT scans or MRI, can be used together with contrast agents to visualize BBB permeability. Functional imaging, such as PET or SPECT, may also be used.

The VLP according to the invention can be administered via various routes, including oral, dermal, nasal administration or pulmonary routes or parenteral injection (i.v., s.c., i.m.). Particularly preferred are dosage forms which allow a systemic effect of the lysosomal enzyme or the expression vector encoding a lysosomal enzyme. In a specific embodiment the VLP according to the invention is administered orally or parenterally, in particular intravenously.

In case the application of the VLP according to the invention leads to an unwanted immune reaction, measured for example by the upregulation of inflammatory cytokines and/or surface molecules on immune cells, it may be necessary to additionally apply an immunosuppressant to reduce the activation or efficacy of the immune system.

In a preferred embodiment, the VLP according to the invention, after administration to the subject to be treated, in particular a human, can be detected in the CNS in less than 10 days, preferably in less than 5 days, more preferably in less than 3 days after administration.

In another preferred embodiment, the lysosomal enzyme has a therapeutically effective enzyme activity for at least 10 days, preferably for at least 20 days, more preferably for at least 30 days. The therapeutically effective enzyme activity can be measured by the enzyme activity which leads to a therapeutic effect, i. e. to at least an alleviation or mitigation of a disease symptom.

It is particularly advantageous that the therapeutically effective enzyme activity preferably at the target site is maintained for at least 10 days, preferably for at least 20 days in order to extend the effective period when the lysosomal enzyme exerts its activity. Thereby, the number or frequency of injections of VLP according to the present invention can be limited.

In the present invention, the lysosomal enzyme preferably is arylsulfatase A.

In particular, the lysosomal enzyme is a human enzyme. Hence, in a particularly preferred embodiment, the lysosomal enzyme is human arylsulfatase A.

In one embodiment, the lysosomal enzyme comprises an amino acid sequence which is at least 80 %, more preferably at least 90 % identical to the amino acid sequence according to SEQ ID NO: 1 over its entire length, preferably has the amino acid sequence of SEQ ID NO: 1.

In one embodiment, the lysosomal enzyme comprises a nucleotide sequence which is at least 70 %, more preferably at least 80 %, more preferably at least 90 % identical to the nucleotide sequence of SEQ ID NO: 2 over its entire length, preferably is the nucleotide sequence of SEQ ID NO: 2.

The sequences with SEQ ID NOs 1 and 2 pertain to the enzyme human arylsulfatase A and are shown in the table below:

**Table 1: Amino acid and nucleotide sequences of the enzyme human arylsulfatase A.**

| **Protein / DNA** | **Sequence** | **Source** | **SEQ ID NO:** |
|---|---|---|---|
| **Protein** | | human | 1 |
| **DNA** | ATGTCCATGGGGGCACCGCGGTCCCTCC TCCTGGCCCTGGCTGCTGGCCTGGCCGT TGCCCGTCCGCCCAACATCGTGCTGATCT TTGCCGACGACCTCGGCTATGGGACCT GGGCTGCTATGGGCACCCCAGCTCTACC ACTCCCAACCTGGACCAGCTGGCGGCGG GAGGGCTGCGGTTCACAGACTTCTACGT GCCTGTGTCTCTGTGCACACCCTCTAGG GCCGCCCTCCTGACCGGCCGGCTCCCG GTTCGGATGGGCATGTACCCTGGCGTCC TGGTGCCCAGCTCCCGGGGGGGCCTGC CCCTGGAGGAGGTGACCGTGGCCGAAGT CCTGGCTGCCCGAGGCTACCTCACAGGA ATGGCCGGCAAGTGGCACCTTGGGGTGG GGCCTGAGGGGGCCTTCCTGCCCCCCCA TCAGGGCTTCCATCGATTTCTAGGCATCC CGTACTCCCACGACCAGGGCCCCTGCCA | human | 2 |
| | GAACCTGACCTGCTTCCCGCCGGCCACT CCTTGCGACGGTGGCTGTGACCAGGGCC TGGTCCCCATCCCACTGTTGGCCAACCTG TCCGTGGAGGCGCAGCCCCCCTGGCTGC CCGGACTAGAGGCCCGCTACATGGCTTT CGCCCATGACCTCATGGCCGACGCCCAG CGCCAGGATCGCCCCTTCTTCCTGTACTA TGCCTCTCACCACACCCACTACCCTCAGT TCAGTGGGCAGAGCTTTGCAGAGCGTTC AGGCCGCGGCCATTTGGGGACTCCCTG ATGGAGCTGGATGCAGCTGTGGGACCC TGATGACAGCCATAGGGGACCTGGGGCT GCTTGAAGAGACGCTGGTCATCTTCACTG CAGACAATGGACCTGAGACCATGCGTAT GTCCCGAGGCGGCTGCTCCGGTCTCTTG CGGTGTGGAAAGGGAACGACCTACGAGG GCGGTGTCCGAGAGCCTGCCTTGGCCTT CTGGCCAGGTCATATCGCTCCCGGCGTG ACCCACGAGCTGGCCAGCTCCCTGGACC TGCTGCCTACCCTGGCAGCCCTGGCTGG GGCCCCACTGCCCAATGTCACCTTGGAT GGCTTTGACCTCAGCCCCCTGCTGCTGG GCACAGGCAAGAGCCCTCGGCAGTCTCT CTTCTTCTACCCGTCCTACCCAGACGAGG TCCGTGGGGTTTTTGCTGTGCGGACTGG AAAGTACAAGGCTCACTTCTTCACCCAGG GCTCTGCCCACAGTGATACCACTGCAGA CCCTGCCTGCCACGCCTCCAGCTCTCTG ACTGCTCATGAGCCCCCGCTGCTCTATGA CCTGTCCAAGGACCCTGGTGAGAACTAC AACCTGCTGGGGGGTGTGGCCGGGGCC ACCCCAGAGGTGCTGCAAGCCCTGAAAC AGCTTCAGCTGCTCAAGGCCCAGTTAGA CGCAGCTGTGACCTTCGGCCCCAGCCAG GTGGCCCGGGGCGAGGACCCCGCCCTG CAGATCTGCTGTCATCCTGGCTGCACCCC CCGCCCAGCTTGCTGCCATTGCCCAGAT CCCCATGCCTGA | | |

Preferably, the expression vector encoding the lysosomal enzyme has a size of less than 7 kb, preferably less than 6 kb. The associating of the VLP with the expression vector is more efficient when the size of the expression vector is relatively small, preferably less than 6 kb or even less than 5 kb.

In a preferred embodiment, the expression vector has a promoter selected from the group comprising CMV and CAG.

The CAG promoter is particularly advantageous because it allows a long-lasting expression. The CAG promoter also inhibits unwanted immune reactions so that the additional application of immunosuppressants may be reduced or even completely avoided.

The CMV promoter is preferred if a stronger and shorter expression is needed.

The need of a long-lasting or short expression may vary depending on the disease, the stage of the disease and the subject to be treated. A long-lasting expression of the lysosomal enzyme allows a long-lasting effect which is beneficial if there is no or only little residual activity of the lysosomal enzyme in the subject. In case there is residual activity of the lysosomal enzyme, a shorter expression may be sufficient. Likewise, the need of a light or strong expression may vary.

Preferably, the lysosomal enzyme is expressed, preferably at the target site, for at least 1 h, preferably for at least 5 h, more preferably for at least 12 h, more preferably for at least 1 day, more preferably for at least 3 days, more preferably for at least 1 week, more preferably for at least 1 month, more preferably for at least 3 months, more preferably for at least 6 months, more preferably for at least 9 months, more preferably for at least 1 year, more preferably for at least 1.5 years, more preferably for at least 2 years.

Most preferably, the lysosomal enzyme is expressed at the target site for at least 1 month.

In one embodiment therefore, the lysosomal enzyme is detectable at the target site for at least 1 h, preferably for at least 5 h, more preferably for at least 12 h, more preferably for at least 1 day, more preferably for at least 3 days, more preferably for at least 1 week, more preferably for at least 1 month, more preferably for at least 3 months, more preferably for at least 6 months, more preferably for at least 9 months, more preferably for at least 1 year, more preferably for at least 1.5 years, more preferably for at least 2 years.

Most preferably, the lysosomal enzyme is detectable at the target site for at least 1 month.

Preferably, the expression in the target cell is transient. A transient expression does not exclude that the expression is long-lasting. In a preferred embodiment, the expression is long-lasting and transient.

In a preferred embodiment, the expression vector is a plasmid.

In a particularly preferred embodiment, the plasmid is free of antibiotic resistance genes. This is advantageous because it allows culturing such plasmids without antibiotics. This is in particular critical for clinical use because the injection of antibiotics may lead to sensitization or to anaphylactic shocks. A "pFAR" plasmid is for example a plasmid without antibiotic resistance genes. Preferably, a pFAR plasmid is used. A pFAR plasmid allows a long-lasting and stable expression without the need to use antibiotics.

In another preferred embodiment, a "pNL" plasmid or a "pSF" plasmid is used.

The expression "pFAR plasmid", "pNL plasmid" or "pSF plasmid" means that a plasmid with the backbone of a pFAR plasmid, a pNL plasmid or a pSF plasmid is used for cloning the promoter and the lysosomal enzyme of interest into the backbone.

A pFAR plasmid is for example described in US 8,440,455 B2. A particularly preferred pFAR plasmid is pFAR4, the backbone of which is disclosed as SEQ ID NO: 21 in US 8,440,455 B2, the sequence is herein disclosed as SEQ ID NO: 7. The construction of the "pFAR1" plasmid and the optimized "pFAR4" plasmid is disclosed in columns 17 and 18 of US 8,440,455 B2.

The pFAR plasmid preferably comprises a CMV or a CAG promoter. The pNL plasmid preferably comprises a CMV promoter. The pSF plasmid preferably comprises a CAG promoter.

In a preferred embodiment, the expression vector is pFAR-CMV-ASA, thus a pFAR backbone with a CMV promoter and encoding the human ASA gene. In another preferred embodiment, the expression vector is pFAR-CAG-ASA. In another preferred embodiment, the expression vector is pNL-CMV-ASA. The expression vector pFAR-CAG-ASA is particularly preferred.

It has been shown in the present invention that human ASA can be expressed in glioblastoma cells using different expression vectors.

In a preferred embodiment, if the cargo is an expression vector encoding a lysosomal enzyme the treatment of the lysosomal storage disease, preferably MLD, is effected by the transient expression of the lysosomal enzyme gene in the target cell, preferably an oligodendrocyte, of the subject to be treated.

Subjects to be treated by the VLP according to the present invention are e. g. patients suffering from a lysosomal storage disease or subjects expected to suffer from a lysosomal storage disease, for example because they encompass a genetic mutation which is known to affect a lysosomal enzyme and thus leads to a lysosomal storage disease.

In a preferred embodiment, the lysosomal storage disease is one which is associated or potentially associated with a neurologic deficit of the subject. "Potentially associated" means that the normal course of development of the disease eventually leads to a neurologic deficit although the subject may not have a neurologic deficit yet. Preferably, the subject to be treated has a neurologic deficit.

A "neurologic deficit" according to the invention refers to abnormal or altered body function due to an altered function of brain, spinal cord, muscles, or nerves or other parts of the CNS. Examples include abnormal reflexes, disturbance of speech or balance, decreased sensation, mental function problems, vision changes, walking problems or weakness of arms or legs. Reference is a healthy subject with normal reflexes, an ability to speak, normal sensation, balance, mental function, vision and walking and strength of arms and legs.

A lysosomal storage disease which is associated or potentially associated with a neurologic deficit of the subject is for example MLD. In a very preferred embodiment, the lysosomal storage disease is MLD.

If the lysosomal storage disease is associated or potentially associated with a neurologic deficit of the subject, the target of the VLP according to the invention is preferably the CNS. More specifically, the target cell is an astrocyte, an oligodendrocyte, a neuron or microglia, in particular an oligodendrocyte.

Different mouse models of MLD exist which can be used to study the disease and possible therapeutics:
ASA(-/-) mice have a double knock-out of the ASA gene. Deficient animals store the sphingolipid cerebroside-3-sulfate in various neuronal and non-neuronal tissues. The storage pattern is comparable to that of affected humans, but gross defects of white matter are not observed up to the age of 2 years. Neurologic examination at 12 to 14 months of age reveals significant impairment of neuromotor coordination (Hess et al., Proc Natl Acad Sci U S A. 1996;93(25):14821-6).
ASA(-/-)/CST (alternatively ASA(-/-)/Gal3St1) are ASA(-/-) mice overexpressing the sulfatide-synthesizing enzyme Cerebrosid sulfotransferase (CST) (alternatively galactose-3-*O*-sulfotransferase-1 (Gal3St1)) in myelinating cells. These mice display a significant increase in sulfatide storage in brain and peripheral nerves. Mice older than 1 year develop severe neurological symptoms (Ramakrishnan et al., J Neurosci. 2007;27(35):9482-90).
In ASA(-/-)/CGT mice neural cells overexpress the sulfatide synthesizing enzyme UDP-galactose:ceramide galactosyltransferase (CGT). These mice show increased sulfatide storage in myelin-forming cells, resulting in axonal degeneration and development of neurological symptoms similar to MLD. (Eckhardt et al., J Neurosci. 2007;27(34):9009-21).
ASA(-/-)/hASAc69s mice show immunotolerance to hASA (expression of inactive hASA) and maintenance of the MLD-like phenotype of ASA(-/-) mice. Enzyme replacement therapy has been studied with this model (Matzner et al., Mol Med. 2007;13(9-10):471-9).
ASA(-/-)/CST/hASAc69s mice are double-transgenic mASA mice with immunotolerance to hASA (expression of inactive hASA) and supranormal rates of sulfatide synthesis (cerebroside sulfotransferase (CST) overexpression). Enzyme replacement therapy has been studied with this model (Matthes et al., Hum Mol Genet. 2012;21(11):2599-609).

The VLP of the invention is preferably derived from John Cunningham virus (JCV). The "JC virus" or John Cunningham virus (JCV; NCBI Taxonomy 10632) is a human polyomavirus. JCV is of an icosahedral symmetry, has a diameter of about 45 nm and consists of 72 VP1 pentamers. Small numbers of the structural proteins VP2 and VP3 are also present.

A "virus-like particle" (VLP) in the context of the present invention is defined as a replication-deficient particle with a hull (also termed capsid) composed of viral structural proteins or modified viral structural proteins or proteins derived from viral structural proteins. As explained above, the VLP as such does not comprise genetic material. The VLP according to the invention is preferably derived from a human polyoma virus, preferably JCV, i.e. its hull is preferably composed of viral structural proteins or modified viral structural proteins or proteins derived from viral structural proteins VP1, VP2 and VP3 from JCV. In a preferred embodiment, the VLP according to the invention is composed of VP1 proteins of JC virus.

In a particularly preferred embodiment of the invention the only viral structural protein in the hull of the VLP according to the invention is a VP1 protein. In the most preferred embodiment the hull of the VLP according to the invention consists of VP1 proteins, i.e. the hull does not contain any other protein.

The viral structural proteins, in particular the VP1, assemble into pentameric structures (pentamers). According to the invention, the VLP hull according to the invention preferably is composed of several VP1 proteins, in particular several VP1 pentamers, especially 72 VP1 pentamers.

A "pentamer" in the context of the invention is a structure which is formed when five polypeptides, for example VP1 proteins, assemble. The assembly into a pentamer may be due to the formation of covalent or non-covalent bonds between the polypeptides. The polypeptides typically form a ring-shaped structure, having pentagonal symmetry. In a pentamer, each polypeptide subunit preferably interacts with two adjacent subunits.

A "peptide" according to the present invention may be composed of any number of amino acids of any type, preferably naturally occurring amino acids, which preferably are linked by peptide bonds. In particular, a peptide comprises at least 3 amino acids, preferably at least 5, at least 7, at least 9, at least 12 or at least 15 amino acids. There is no upper limit for the length of a peptide. However, preferably a peptide according to the invention does not exceed a length of 500 amino acids, preferably 400, 300, 250, 200, 150 or 120 amino acids. A peptide exceeding about 10 amino acids may also be termed a "polypeptide".

The structural proteins of the VLP according to the invention, in particular the VP1, are preferably identical to or derived from the native structural proteins of JCV. "Modified or derived" encompasses the insertion, deletion or substitution of one or more amino acids while retaining the function of VP1 to assemble into a capsid.

In one embodiment, the native (JCV) structural protein can be modified in order to optimize the VLP according to the invention with regard to its production, its cellular targeting profile and specificity or its intracellular targeting profile or specificity. Modification or derivation can comprise a codon optimization of the nucleotide sequence encoding the structural protein, in particular the VP1, to enhance protein translation.

The terms "VP1" or "virus protein 1" according to the present invention refer to a protein which is capable of assembling into a capsid and which is preferably identical to or is derived from the natural (native) VP1 of the JCV.

The term "VP1" according to the invention encompasses a protein which has an amino acid sequence identity with the amino acid sequence according to SEQ ID NO: 3 of at least 80%, more preferably at least 85%, more preferably at least 90%, more preferably at least 95%, more preferably at least 97%, more preferably at least 98% or at least 99% over this sequence. In a most preferred embodiment of the invention the VP1 has the amino acid sequence according to SEQ ID NO: 3.

The term "VP1" according to the invention also encompasses fractions of the native VP1. Preferably, said fractions of VP1 comprise at least acids 32 to 316 of the amino acid sequence according to SEQ ID NO: 3 or a derivative thereof having an identity with the amino acid sequence from amino acid position 32 to 316 of SEQ ID NO: 3 of at least 80%, more preferably at least 85%, more preferably at least 90%, more preferably at least 95%, more preferably at least 97%, more preferably at least 98% or at least 99% over this sequence.

In another embodiment of the invention the VP1 has an amino acid sequence which is at least 80 %, more preferably at least 85 %, more preferably at least 90 %, more preferably at least 95 %, more preferably at least 99 % identical to the amino acid sequence according to SEQ ID NO: 4 over its entire length. In one embodiment, the amino acid sequence of VP1 is identical to the amino acid sequence of SEQ ID NO: 4.

In one embodiment, the nucleotide sequence of the VP1 protein is at least 70 %, more preferably at least 80 %, more preferably at least 90 % identical to the nucleotide sequence of SEQ ID NO: 5 over its entire length, preferably is the nucleotide sequence of SEQ ID NO: 5.

In another embodiment of the invention the nucleotide sequence of the VP1 protein is at least 70 %, more preferably at least 80 %, more preferably at least 90 % identical to the nucleotide sequence of SEQ ID NO: 6 over its entire length. In one embodiment, the nucleotide sequence of the VP1 protein is identical to the nucleotide sequence of SEQ ID NO: 6.

The sequences are depicted in Table 2.

**Table 2: Amino acid and nucleotide sequences of the VP1 protein.**

| **Protein / DNA** | **Sequence** | **Source** | **SEQ ID NO:** |
|---|---|---|---|
| **Protein** | MAPTKRKGEPKDPVQVPKLLIRGGVEVLE VKTGVDSITEVECFLTPEMGDPDEHLRGF SKSISISDTFESDSPNRDMLPCYSVARIPL PNLNEDLTCGNILMWEAVTLKTEVIGVTSL MNVHSNGQATHDNGAGKPVQGTSFHFFS VGGEALELQGVVFNYRTKYPDGTIFPKNA TVQSQVMNTEHKAYLDKNKAYPVECWVP DPTRNENTRYFGTLTGGENVPPVLHITNT ATTVLLDEFGVGPLCKGDNLYLSAVDVCG MFTNRSGSQQWRGLSRYFKVQLRKRRV KNPYPISFLLTDLINRRTPRVDGQPMYGM DAQVEEVRVFEGTEELPGDPDMMRYVDK YGQLQTKML | derived from JCV | 3 |
| **Protein** | MAPTKRKGERKDPVQVPKLLIRGGVEVLE VKTGVDSITEVECFLTPEMGDPDEHLRGF SKSISISDTFESDSPSKDMLPCYSVARIPLP NLNEDLTCGNILMWEAVTLKTEVIGVTSLM NVHSNGQAAHDNGAGKPVQGTSFHFFSV GGEALELQGVVFNYRTKYPDGTIFPKNAT VQSQVMNTEHKAYLDKNKAYPVECWVPD PTRNENTRYFGTLTGGENVPPVLHITNTA TTVLLDEFGVGPLCKGDNLYLSAVDVCGM | wildtype JCV (AFH571 94.1) | 4 |
| | FTNRSGSQQWRGLSRYFKVQLRKRRVKN PYPISFLLTDLINRRTPRVDGQPMYGMDA QVEEVRVFEGTEELPGDPDMMRYVDRYG QLQTKML | | |
| **DNA** | ATGGCTCCCACCAAGCGCAAGGGCGAG CCCAAGGACCCCGTGCAAGTGCCCAAG CTGCTGATCCGTGGTGGTGTCGAGGTG CTGGAAGTCAAGACCGGCGTGGACTCC ATTACCGAGGTGGAGTGCTTCCTCACCC CCGAGATGGGTGACCCTGACGAGCACC TGAGGGGCTTCTCCAAGTCCATCTCCAT CTCCGACACCTTCGAGTCCGACTCCCC CAACCGTGACATGCTGCCCTGCTACTCC GTGGCTCGTATCCCCCTGCCCAACCTG AACGAGGACCTGACTTGCGGCAACATC CTGATGTGGGAGGCTGTGACCCTCAAG ACCGAGGTCATCGGCGTGACTTCCCTG ATGAACGTGCACTCCAACGGCCAGGCT ACCCACGACAACGGTGCTGGCAAGCCC GTGCAGGGAACCTCCTTCCACTTCTTCT CCGTGGGTGGCGAGGCTCTGGAACTCC AGGGCGTGGTGTTCAACTACCGTACCAA GTACCCCGACGGCACCATCTTCCCCAA GAACGCTACTGTGCAGTCCCAAGTGATG AACACCGAGCACAAGGCTTACCTGGAC AAGAACAAGGCCTACCCCGTGGAGTGC TGGGTGCCCGACCCCACCCGTAACGAG AACACCCGTTACTTCGGCACCCTGACCG GTGGAGAGAACGTGCCCCCCGTGCTGC ACATCACCAACACCGCTACCACCGTGCT GCTGGACGAGTTCGGTGTCGGTCCCCT GTGCAAGGGCGACAACCTGTACCTGTC CGCTGTGGACGTGTGCGGCATGTTCAC CAACCGTTCCGGTTCCCAGCAGTGGCG TGGCCTGTCCCGCTACTTCAAGGTGCA GCTGCGCAAGCGTCGTGTGAAGAACCC CTACCCTATCTCCTTCCTGCTGACCGAC CTGATCAACCGTCGTACCCCTCGTGTGG ACGGCCAGCCCATGTACGGCATGGACG CTCAGGTGGAAGAGGTCCGCGTGTTCG AGGGCACCGAGGAATTGCCCGGCGACC CCGACATGATGCGTTACGTGGACAAGTA CGGCCAGCTCCAGACCAAGATGCTGTA A | derived from JCV | 5 |
| **DNA** | ATGGCCCCAACAAAAAGAAAAGGAGAAA GGAAGGACCCCGTGCAAGTTCCAAAAC TTCTTATAAGAGGAGGAGTAGAAGTTCT AGAAGTTAAAACTGGGGTTGACTCAATT ACAGAGGTAGAATGCTTTTTAACTCCAG AAATGGGTGACCCAGATGAGCATCTTAG GGGTTTTAGTAAGTCAATATCTATATCAG ATACATTTGAAAGTGACTCCCCAAATAG GGACATGCTTCCTTGTTACAGTGTGGCC AGAATTCCACTACCCAATCTAAATGAGG ATCTAACCTGTGGAAATATACTCATGTG GGAGGCTGTGACCTTAAAAACTGAGGTT ATAGGGGTGACAAGTTTGATGAATGTGC ACTCTAATGGGCAAGCAACTCATGACAA TGGTGCAGGGAAGCCAGTGCAGGGCAC CAGCTTTCATTTTTTTCTGTTGGGGGG GAGGCTTTAGAATTACAGGGGGTGCTTT TTAATTACAGAACAAAGTACCCAGATGG AACAATTTTTCCAAAGAATGCCACAGTG CAATCTCAAGTCATGAACACAGAGCACA AGGCGTACCTAGATAAGAACAAAGCATA TCCTGTTGAATGTTGGGTTCCTGATCCC ACCAGAAATGAAAACACAAGATATTTTG GGACACTAACAGGAGGAGAAAATGTTCC TCCAGTTCTTCATATAACAAACACTGCCA CAACAGTGTTGCTTGATGAATTTGGTGT TGGGCCACTTTGCAAAGGTGACAACTTA TACTTGTCAGCTGTTGATGTCTGTGGCA TGTTTACAAACAGGTCTGGTTCCCAGCA GTGGAGAGGACTCTCCAGATATTTTAAG GTGCAGCTAAGGAAAAGGAGGGTTAAA AACCCCTACCCAATTTCTTTCCTTCTTAC TGATTTAATTAACAGAAGGACTCCTAGA GTTGATGGGCAGCCTATGTATGGCATG GATGCTCAAGTAGAGGAGGTTAGAGTTT TTGAGGGAACAGAGGAGCTTCCAGGGG ACCCAGACATGATGAGATACGTTGACAA ATATGGACAGTTGCAGACAAAAATGCTG TAA | wildtype JCV (J02226) | 6 |

In a preferred embodiment of the invention, the VP1 has an amino acid sequence which is at least 90 % identical to the amino acid sequence according to SEQ ID NO: 3 over its entire length.

In a preferred embodiment of the invention, the nucleotide sequence of the VP1 protein is at least 80 % identical to the nucleotide sequence of SEQ ID NO: 5 over its entire length.

The structural proteins, preferably VP1, can be expressed in, for example, *E. coli* or in insect cells. According to a preferred embodiment of the invention, the structural proteins, preferably VP1, are expressed in insect cells. This is advantageous because the expression in insect cells leads to fewer modifications, such as post-translational modifications, compared with the wildtype protein, for example from JCV, than the expression in *E. coli.*

The VLP according to the invention can furthermore comprise in the capsid one or several additional heterologous proteins, i.e. proteins which are not identical to or derived from the source of the VP1, e. g. JCV. For example, a heterologous protein can be anchored in the capsid, i.e. at least part of this protein being preferably accessible from the outside. In principle any protein is suitable as such a heterologous protein as long as the heterologous protein can be incorporated into the capsid and does not interfere substantially with the assembly of the VLP according to the invention.

The VLP according to the invention is associated with a cargo, i. e. with a lysosomal enzyme or an expression vector encoding a lysosomal enzyme. This means that the cargo is reversibly bound to the VLP. This can e.g. either be due to a physicochemical interaction with or attachment to any part of the capsid or by incorporation of the cargo into the capsid. The incorporation can be complete or incomplete. In a particular preferred embodiment of the invention the major part of the total amount of the cargo is fully incorporated into the capsid. Most preferred is that the cargo is fully encapsulated in the capsid of the VLP according to the invention.

In the context of the invention, the expression that the "VLP comprises the cargo" is used synonymously with the expression that the VLP is "associated with the cargo". The association of the VLP and the cargo can be the result of "loading" or "packing" the VLP with the cargo.

"Loading" means any process which leads to the association of VLP and cargo, e.g. by osmotic shock or by assembly of VP1 or VP1 pentamers into VLP together with the cargo. "Loaded VLP" are the VLP resulting from this process. The term "packing" relates to the process of loading the VLP via assembly of VP1 or VP1 pentamers into VLP together with the cargo. The VLP resulting therefrom are termed "packed" VLP.

The term "cargo" is used, in the context of the present invention, for a lysosomal enzyme or an expression vector encoding a lysosomal enzyme.

In a special embodiment of the invention, the VLP according to the invention is part of a pharmaceutical composition for use in a method for the treatment of a lysosomal storage disease in a subject, wherein the pharmaceutical composition further comprises a pharmaceutically acceptable carrier, and/or excipient.

In a preferred embodiment, the pharmaceutical composition comprises VLP according to the invention, a salt and a buffer and has a pH of between 7.0 and 8.0, preferably around 7.5. The pharmaceutical composition preferably comprises:
a. 120 mM to 170 mM NaCl, preferably 150 mM NaCl,
b. 1 to 5 mM CaCl₂, preferably 2 mM CaCl₂, and
c. 5 to 30 mM Tris-HCl, preferably 10 to 25 mM Tris-HCl, more preferably 10 mM Tris-HCl.

This pharmaceutical composition allows handling the VLP according to the invention under physiological conditions. Under these conditions the VLP according to the invention essentially remain intact, preferably they essentially maintain their capsid structure. If loaded with cargo, such as a lysosomal enzyme or an expression vector encoding a lysosomal enzyme, the VLP according to the invention essentially remain associated with the cargo. The pharmaceutical composition is especially suitable as a pharmaceutical composition for the intravenous administration of the VLP according to the invention to a subject, in particular to a human.

In a further aspect, the invention relates to an expression vector having a coding region encoding a lysosomal enzyme, preferably human arylsulfatase A, a promoter selected from the group comprising CAG and CMV, preferably being CAG, and having a size of less than 7 kb, preferably less than 6 kb.

In a preferred embodiment, the lysosomal enzyme encoded by the expression vector comprises a nucleotide sequence which is at least 70 %, more preferably at least 80 %, more preferably at least 90 % identical to the nucleotide sequence of SEQ ID NO: 2 over its entire length, preferably is the nucleotide sequence of SEQ ID NO: 2.

Preferably, the expression vector encodes human arylsulfatase A, comprises the promoter CAG and has a size of less than 6 kb.

In case the VLP according to the invention are not immediately administered after manufacture, they can be stored, preferably in liquid nitrogen.

The VLP according to the invention can be characterized according to standard methods, for example by a Bradford assay, HA, DLS, nDSF, HPLC-SEC, AF4, TEM.

The invention also relates to a method of treating a lysosomal storage disease, in particular MLD, with the VLP according to the invention. The method of treating preferably comprises the step of administering the VLP to a subject in need thereof. The invention also relates to the use of the VLP according to the invention for the manufacture of a medicament for the treatment of a lysosomal storage disease, in particular MLD. The method of treatment preferably does not comprise a step of increasing the permeability of the BBB of the subject to be treated. The VLP of the invention, preferably, is administered to a patient who has not received any chemical or physical treatment for impairing or disrupting the BBB.

The invention also relates to VLP according to the invention which are used to deliver a lysosomal enzyme or an expression vector encoding a lysosomal enzyme across the BBB to the CNS, in particular to cells of the CNS, for example astrocytes, oligodendrocytes, neurons and microglia.

Importantly, the crossing of the BBB by VLP according to the invention enables the VLP to exhibit is function of targeting specific cell populations within the brain, i.e. deliver a lysosomal enzyme or an expression vector encoding a lysosomal enzyme to target cells, preferably astrocytes, oligodendrocytes, neurons and microglia. In the context of the invention said VLP comprises a delivery to and/or into the target cells.

In a further embodiment, the invention relates to a method of associating a VLP with an expression vector encoding a lysosomal enzyme, preferably human arylsulfatase A, wherein the method comprises the following steps:
- providing VLP, in particular derived from JCV
- exposing the VLP to conditions dissociating the VLP into pentamers
- exposing the pentamers to the expression vector in a ratio of VLP to expression vector of 1 to 0.5 to 1 to 0.1, preferably in a ratio of 1 to 0.2, and to conditions inducing the pentamers to assemble into a VLP associated with the expression vector
- optionally purifying the VLP
- performing a dialysis step.

In another preferred embodiment, a method of associating a VLP with a lysosomal enzyme, preferably human arylsulfatase A, or an expression vector encoding a lysosomal enzyme, preferably human arylsulfatase A, is provided, wherein the method comprises the following steps:
a) providing a composition comprising VP1 proteins,
b) exposing the VP1 proteins of the composition of a) to conditions inducing the VP1 to assemble into VLP,
c) exposing the VLP of the composition of b) to conditions disassembling the VLP into pentamers,
d) exposing the pentamers of the composition of c) to conditions inducing the pentamers to reassemble into VLP
e) exposing the VLP of the composition of d) to conditions disassembling the VLP into pentamers,
f) exposing the pentamers of the composition of e) to the lysosomal enzyme or the expression vector to conditions inducing the pentamers to assemble into a VLP associated with the lysosomal enzyme or the expression vector.

The ratio of VLP to expression vector (i. e. the packaging ratio) can be varied depending on the specific need. For example, the efficiency of VLP formation or gene expression may be dependent on the ratio. Preferably, a ratio of VLP to expression vector of 1 to 0.5 to 1 to 0.1, more preferably of 1 to 0.2 is used. The person skilled in the art will tailor the ratio to the specific expression vector, preferably plasmid, and desired usage. A packaging ratio of 1 to 0.2 is preferred.

In the context of the present invention, and for the ease of explanation, the VLP resulting from step b) may also be termed "pVLP" (primary VLP). The VLP resulting from step d) may also be termed "rVLP" (reassembled VLP). The VLP as the result of step f) may also be termed "cVLP" (cargoVLP). According to the present invention, cargo is a lysosomal enzyme, preferably human arylsulfatase A, or an expression vector encoding a lysosomal enzyme, preferably human arylsulfatase A.

In another aspect of the invention, the invention relates to VLP obtainable by the method above.

In another aspect of the invention, the invention relates to a composition comprising VLP according to the invention derived from JCV characterized by one or more of the following parameters:
a. a polydispersity index (PDI) of less than 0.3, preferably less than 0.2, preferably less than 0.1, more preferably in a range between 0.01 and 0.09,
b. at least 70 % of VLP with an average diameter from 20 nm to 70 nm, preferably of 30 nm to 70 nm, more preferably of 35 nm to 65 nm, more preferably of 40 to 60 nm,
c. a VLP content within the composition of at least 80 % (v/v), preferably at least 85 % (v/v), preferably at least 90 % (v/v), preferably at least 95 % (v/v).

In another aspect, the invention relates to a drug delivery system obtainable by the method according to the invention. The drug delivery system can be used in a method of therapy and/or diagnosis, preferably for the treatment of neurological disorders, namely a lysosomal storage disease, in particular MLD. Hence the invention also relates to a method of treatment a disorder, in particular a CNS disease, with the drug delivery system according to the invention. The method of treatment preferably comprises the step of administering the drug delivery system to a subject in need thereof.

The drug delivery system preferably has an improved efficacy. The VLP according to the invention can cross the BBB without a prior increase of the permeability of the BBB. Hence, the drug delivery system of the invention can be used in a method of treatment of a CNS disease, wherein the method does not comprise a step of increasing the permeability of the BBB of the subject to be treated. The drug delivery system of the invention, preferably, is administered to a patient who has not received any chemical or physical treatment for impairing or disrupting the BBB.

In yet a further aspect of the invention a composition comprising VLP is provided, which has least one, preferably all, of the following characteristics ("target parameters"):

**Table 3: Preferred characteristics of the VLP and the VLP-containing composition according to the invention.**

| AUC = area under the curve | | |
|---|---|---|
| **Methods for evaluation** | **Measurement** | **Target Parameters** |
| **Transmission Electron Microscopy (TEM)** | VLP per grid mesh | >50 particles |
| | Shape of particles | round and enclosing |
| | Diameter of particles | 40-50 nm |
| | Aggregates | Not visible |
| **SDS PAGE and Western Blot (WB)** | VP1 band | 40 kDa band is observed |
| | VP1 degradation | Minor or no degradation |
| **Dynamic Light Scattering (DLS)** | PDI | <0.2 |
| | Single peak (volume based distribution) | Yes |
| | Z-average diameter | 40-50 nm |
| | Other peaks (volume based distribution) | Not detectable |
| **Bioanalyzer** | VP1 purity (40 kDa) | >90% |
| **Thermal Shift Assay (TSA)** | Major melting peak (in Tris-HCI-Buffer) | >57°C |
| | Minor melting peaks | Not detectable |
| **Nano Differential Scanning Fluorometry (nDSF)** | Major inflection peak | >69°C |
| | Minor inflection peaks | Not detectable |
| **Size Exclusion HPLC (SE-HPLC)** | Aggregates | <5% of total AUC |
| | Other impurities | <5% of total AUC |
| **Field Flow Fractionation (FFF-MALS)** | Concentration of particles | >1.0 x 10¹¹ VLP/mL |
| | Size of particles | 40-50 nm |
| | Aggregates | <5% of total AUC |
| | Other impurities | <5% of total AUC |

In one embodiment, the VLP according to the invention show a major inflection peak in nDSF analyses of >67°C.

In one embodiment, AF4 analyses of the VLP according to the invention reveal less than 20 % aggregates and less than 15 % tinies. At least 70 % are VLP of 40 to 50 nm in size.

The drug delivery system of the invention can be administered via various routes, including oral, dermal, nasal or pulmonary routes or injection. Particularly preferred are dosage forms which allow a systemic effect of the pharmaceutical product. In a specific embodiment the drug delivery system of the invention is administered orally or parenterally, in particular intravenously.

In the context of the invention, the term "drug delivery system" refers to a composition for administering a pharmaceutical product to a subject in the need thereof, in particular to a human or animal. A drug delivery system, advantageously, enables the delivery of the pharmaceutical product contained therein or attached thereto to a site of interest, preferably in a human or animal. Preferably the delivery is selective for the target, i.e. more of the pharmaceutical product is delivered to the target than to other sites of the body or organ.

"A drug delivery system for the CNS" means that the drug delivery system selectively targets the CNS.

According to the invention the expression "exposing" something (e.g. the VP1, pentamers, the VLP) to conditions for affecting something (e.g. inducing the assembly) refers to bringing the material under consideration (e.g. the VP1, the pentamers, the VLP) to conditions which can cause this certain effect (e.g. inducing the assembly). Such exposure may be performed by changing the conditions for the material, e.g. by bringing the material into contact with a different buffer, salt or pH etc. This is possible either by adding something to the composition comprising the material or *vice versa* or by separating the material from the composition and then adding the material to a different composition.

A change of conditions can also be achieved by varying temperature, radiation etc. Naturally, such means for a change of conditions can be combined and/or repeated. Other suitable conditions that induce the desired effect, such as the assembly of the VP1 or pentamers to VLP and/or inducing aggregation of the VLP, are also well known to the skilled person. The same applies to a suitable duration of the exposure to the respective conditions; this can be found out by ordinary means of the skilled person.

The expression "exposing something to conditions for affecting something" does not require the effect to be completed, i. e. not all of the material has to accomplish the effect under consideration. For example, "conditions inducing the pentamers to aggregate" essentially means that the conditions are suitable to induce aggregation. It does not require that indeed all pentamers aggregate.

As used herein, the term "assembly" or "assemble into VLP" means that the structures under consideration (either the VP1 proteins or the pentamers) associate and establish the capsid of the VLP. If the VP1 are used as the starting material the assembly into VLP may include the prior formation of pentamers, meaning that the VP1 proteins may first form pentamers and then form VLP or they may directly assemble into a VLP. The assembly to VLP is reversible.

The term "disassembly", in turn, refers to a process, when the capsid of the VLP at least partially disintegrates into pentameric structures and/or the structural proteins. The disassembly may be induced by increasing the temperature, by adding proteases and/or by decreasing intermolecular interactions used to form the VLP such as intermolecular disulfide bridges (e. g. by adding reducing agents or adding chelating agents). Such conditions may also include stepwise exposure to a condition. For instance, the composition may be contacted with a reducing agent before the temperature is increased.

Methods of inducing the VP1 and/or pentamers to assemble into VLP are generally known to the skilled person (Goldmann et al. (J. Virol. 1999; 73(5): 4465-69); DE 195 43 553 A1). The same applies to the disassembly of VLP into pentamers. The skilled person, hence, is aware of methods for the control of the assembly and disassembly of VLP.

In one embodiment of the invention the concentration of Ca²⁺ ions in the composition containing the VP1 or pentamers is used for the control of the assembly/disassembly of the VLP. For example, in order to induce the assembly, the concentration of free Ca²⁺ ions can be increased. If the disassembly is desired, the concentration of free Ca²⁺ ions can be lowered by adding a chelating agent to the composition.

A further option for inducing the assembly is to increase the concentration of VP1 pentamers in order to facilitate the assembly into VLP, for example by reducing the solvent in the composition comprising the pentamers. This might require an adaption of the concentration of alkaline earth metals, such as Ca²⁺ or Mg²⁺.

Accordingly to a preferred embodiment of the invention, disassembly can be induced be exposing the VLP to conditions under which intermolecular disulfide bridges are reduced, for example by exposing the VLP to reducing conditions. In a preferred embodiment this step is accomplished in the additional presence of a chelating agent. More preferred, the disassembly is induced by exposing the VLP to reducing conditions in the presence of a chelating agent and optionally at an increased temperature.

In a particular embodiment of the invention, the VLP are exposed to a composition comprising DTT and EDTA and/or EGTA, preferably at a temperature of 15°C to 30°C, preferably 20°C to 25°C, most preferably at a temperature of about 23°C.

According to a preferred embodiment of the invention, the pentamers of the composition of c) are exposed to conditions inducing the aggregation thereof. This step is most suitable if performed before step d). In a preferred embodiment, at least 20 % of the material (e. g. the pentamers) aggregates, preferably at least 30 %, more preferably at least 40 %.

It has surprisingly been found that this step can lead to a more homogeneous size distribution of the VLP. This allows for a better quality management and standardization, which is of utmost importance if the VLP are used in a drug delivery system. Accordingly, this additional procedure preferably is part of the quality control requirements of a drug delivery system.

The term "aggregate" means any particulate structure. "Aggregation" means a process leading to aggregates. This process is reversible.

The aggregation of the pentamers or VLP can be determined by an increased average particle size of the VLP in the composition compared to the control. The larger particle size may be determined by standard methods, such as dynamic light scattering (DLS).

According to a particular embodiment of the invention, the aggregation of the pentamers or VP1 can be induced by one or more agents which are known in the art to facilitate precipitation of proteins (precipitation agent). Most preferred, according to the invention, thus is the use of a precipitation agent.

A "precipitation agent" refers to an agent that promotes aggregation of VP1 or pentamers. The concept of precipitation agents generally is known to the person skilled on the art. Precipitation agents are typically used to facilitate the concentration and purification of proteins. Precipitation can be the result of altering the solvation potential of the solvent, more specifically, by lowering the solubility of the protein. The solubility may also be decreased by adjusting the pH of the composition to the isoelectric point of a protein. Furthermore, lowering the temperature of the composition can also decrease the solubility of a protein.

Possible precipitation agents are e.g. polyethylene glycol (PEG), or alcohol, for example ethanol, and salts. The later are known to the skilled person as "agents for salting out".

Preferably, according to the invention, the precipitation agent is a salt. Most preferred are salts comprising ions known as the "Hofmeister series". The Hofmeister series describes the ordering of ions with respect to their hydrophobic effect on a specific protein in terms of their ability to affect the solubility of said protein in solution. Ions exerting a hydrophobic effect on a protein are especially preferred. Herein, such ions are referred to as kosmotropic ions.

A precipitation agent comprising at least one kosmotropic anion or cation is preferred. Preferred anions are selected from the group consisting of citrate (C₆H₅O₇³⁻), phosphate (PO₄³⁻), sulfate (SO₄²⁻), hydrogen phosphate (HPO₄²⁻), dihydrogen phosphate (H₂PO₄-), iodate (IO₃⁻), hydroxide (OH⁻), fluoride (F⁻), bromate (BrO₃⁻) or acetate (CH₃COO⁻) or combinations thereof, the more preferred anions are citrate, phosphate or sulfate, the most preferred anion is sulfate.

Preferred cations are ammonium or quaternary ammonium compounds (NR₄⁺ with R being an alkyl or an aryl group), such as tetramethylammonium ((CH₃)₄N⁺) or dimethylammonium ((CH₃)₂N₂⁺). Further preferred cations are selected from the list comprising potassium (K⁺), caesium (Cs⁺), rubidium (Rb⁺) or lithium (Li⁺) or combinations thereof, particularly preferred are quaternary ammonium compound or ammonium, most preferred is ammonium.

Therefore, the salt preferably comprises an anion and a cation selected from the group consisting of citrate (C₆H₅O₇³⁻), phosphate (PO₄³⁻), sulfate (SO₄²⁻), hydrogen phosphate (HPO₄²⁻), dihydrogen phosphate (H₂PO₄-), iodate (IO₃⁻), hydroxide (OH⁻), fluoride (F⁻), bromate (BrO₃⁻) or acetate (CH₃COO⁻), quaternary ammonium compounds (NR₄⁺) with R being an alkyl or an aryl group, preferably tetramethylammonium ((CH₃)₄N⁺) or dimethylammonium ((CH₃)₂N₂⁺), ammonium (NH₄⁺), potassium (K⁺), caesium (Cs⁺), rubidium (Rb⁺) or lithium (Li⁺), preferably comprising SO₄²⁻ and/or NH₄⁺ or combinations thereof.

According to a preferred embodiment the salt is selected from the group consisting of (NH₄)₂SO₄, K₂SO₄, Na₂SO₄, (NH₄)₂HPO₄, K₂HPO₄ and Na₂HPO₄. The most preferred salt is ammonium sulfate ((NH₄)₂SO₄).

According to the invention, the aggregation of the pentamers can be induced by any means for bringing the pentamers into contact with the precipitation agent, for example by adding the precipitation agent to the composition comprising the pentamers or *vice versa,* namely adding the composition comprising the pentamers to a precipitation agent. Also other means, such as a dialysis so that the precipitation agent reaches the pentamers by diffusion, is possible.

According to one preferred embodiment of the present invention, the aggregation of the pentamers is induced by a dialysis against a composition comprising the precipitation agent, for example a composition comprising ammonium sulfate.

According to a particularly preferred embodiment of the invention, the composition containing the pentamers for aggregation, has an ammonium sulfate concentration between 0.3 to 5 M, preferably up to 4 M, even more preferred is a concentration between 1.8 and 2.2 M. Most preferred is around 2 M.

The step of inducing aggregation of the pentamers preferably has a duration of at least 1 hour, more preferred of at least 5 hours, even more preferred of at least 12 hours, most preferred of at least 16 hours. It is preferred that the step has a duration is less than 24 hours. In a preferred embodiment the duration of this step is between 14 and 19 hours. In a most preferred embodiment the duration of this step is between 16 and 18 hours. During this time the pentamers are in exposed to conditions for inducing aggregation, in particular they are in contact with ammonium sulfate.

After the step of inducing the aggregation of the pentamers, it is advantageous to include into the process of the invention a step of separating the pentamers from the conditions which had been uses for inducing the aggregation. The methods applicable for such a step are not particularly limited; any method known to the skilled person, which allows for the separation of the pentamers from the aggregation inducing conditions is applicable.

In a preferred embodiment of the invention the pentamers are separated from the conditions inducing their aggregation by dialysis. Dialysis can be used if the aggregation of the pentamers is induced by using a precipitation agent. The principle of dialysis can also favorably be applied in order to bring the pentamers into contact with a precipitation agent. Most preferred is, if the method according to the invention includes at least two steps of dialysis: a first dialysis of the composition of step c) against a composition comprising a precipitation agent, and a second dialysis after the induction of aggregation against a composition which is essentially free of the precipitation agent.

The dialysis for separating the pentamers from the precipitation agent preferably is against a composition which is at least similar to physiological conditions. Such a composition preferably comprises a salt and has a pH of 6 to 8.5, preferably of 6.5 to 8.5, more preferably of 7 to 8, most preferably of 7.2 to 7.5, in particular 7.5. The osmolarity of the composition is preferably between 280 and 310 mosmol/l, most preferably 308 mosmol/l. The composition may for example have a saline (sodium chloride) concentration of 0.8 to 0.92 % (w/v), preferably of 0.9 % (w/v).

Separating the pentamers from the conditions which had been used for inducing the aggregation is preferably performed for at least 1 hour, more preferred for at least 5 hours, 12 hours, more preferably for at least 18 hours, more preferably for about 24 hours or longer. Longer time periods are also possible inter alia depending on the concentration of the pentamers which had been induced to aggregate, the composition comprising the pentamers and the nature and concentration of the precipitation agent. In a preferred embodiment, the composition comprising the aggregated pentamers is dialyzed against a composition similar to physiological conditions for about 24 hours.

The composition preferably further contains a buffer. Suitable buffering systems are known to the skilled person. In a preferred embodiment of the invention the composition includes a TRIS buffer, HEPES buffer, a phosphate buffer or a bicarbonate buffer system. Most preferred is a TRIS buffer.

In a most preferred embodiment the composition comprises 10 mM Tris-HCl and 150 mM NaCl and has a pH of 7.5.

In order to facilitate assembly of the pentamers into VLP, the composition may further comprise divalent ions, such as Ca²⁺ Mg²⁺, Ba²⁺, Cu²⁺, Fe²⁺, Zn²⁺ or combinations thereof. Most preferred is Ca²⁺, for example CaCl₂. In a preferred embodiment, the composition comprises 1 to 3 mM CaCl₂, preferably 2 mM CaCl₂.

In a very preferred embodiment of the invention, the composition which is at least similar to physiological conditions comprises 10 mM Tris-HCl, 150 mM NaCl and 2 mM CaCl₂ and has a pH of 7.5.

In yet another aspect of the invention it has surprisingly been found that the storage of VLP (rVLP) is advantageous compared with the storage of pentamers (Figure 14 and 15). If pentamers are stored and subsequently thawed and reassembled, predominantly "tiny" particles form as well as aggregates. These VLP are not suitable for the production of a drug delivery system. VLP, however, that have been dissociated and reassembled after storage form a particularly homogeneous population of adequately sized VLP according to the invention. Thus, in one embodiment of the invention a composition is provided with particles having an average diameter from 20 nm to 70 nm, preferably of 30 nm to 70 nm, more preferably of 35 nm to 65 nm, more preferably of 40 to 60 nm. A homogeneous size distribution is important in order to fulfil quality control requirements.

In a preferred embodiment, the method according to the invention comprises a step of storing the VLP from the composition of step d). Storing the VLP at a temperature of about -80°C to about 4°C at is possible for a duration of at least 10 h, 15 h, 20 h, preferably for at least 24 h. A storage of even more than 3 days is possible.

In a preferred embodiment the VLP are stored at a temperature below 0°C (freezing). Freezing may be performed using different cooling rates. For example "slow" freezing may occur by applying a cooling rate of about -1°C per minute, while fast freezing may be performed by contacting the sample, i. e. the container which comprises the composition, with liquid nitrogen or by placing the sample in a freezer at -80°C.

In a preferred embodiment, storing takes place in a composition comprising a cryoadditive, preferably selected from the group comprising polyols, sugars, inorganic salts, organic salts, amino acids, polymers, extremolytes or derivatives or combinations thereof.

In a preferred embodiment, the inorganic salt comprises a sulfate anion. Preferred salts comprising a sulfate anion are potassium sulfate, sodium sulfate, sodium thiosulfate, magnesium sulfate and ammonium sulfate. Preferably, the inorganic salt is ammonium sulfate.

The amino acid preferably is glycine, glutamine, proline or alanine. A preferred amino acid derivative is betain. Further possible cryoadditives are glycerol, sucrose, DMSO, ectoin or hydroxyectoin.

It has been found that the addition of cryoadditives, in particular the addition of an inorganic salt (such as a salt comprising a sulfate anion, in particular ammonium sulfate) and/or an amino acid derivative (such as betain), is advantageous with respect to the stability and the functionality or efficacy of the VLP (Figure 19). As stated *supra,* an enhanced stability and/or functionality or efficacy is particularly desired when using VLP as a drug delivery system. It was surprising that the addition of cryoadditives to the composition of step d) has an impact on the packed VLP of step f) in terms of stability and functionality or efficacy.

A cryoadditives serves the purpose of protecting biological tissue from freezing damage (i.e. due to ice formation). The cryoadditives usually operate by increasing the solute concentration in cells. However, in order to be suitable for biological use they must easily penetrate and must not be toxic to cells. Such additives are thus suitable to provide milder storing conditions for the pentamers and/or VLP. Cryoadditives can be supplemented to a composition comprising pentamers and/or VLP to be frozen for storage.

According to a particular preferred embodiment of the present invention, the cryoadditive is added to the composition comprising VLP for subsequent freezing after the VLP, preferably rVLP, were assembled using two dialysis steps (two-step reassembly).

Suitable molar concentrations of cryoadditives except for the polyol-based cryoadditives may be 0.5 M, 0.6 M, 0.7 M, 0.8 M, 0.9 M, 1 M, 1.1 M, 1.2, 1.3 M, 1.4 M, 1.5 M, 2 M, 3 M, 4 M, 5 M. Preferentially, these cryoadditives are used at a molar concentration of about 1 M, preferably at a molar concentration of 1 M.

The polyol-based cryoadditive may be used at molar concentrations of at least 0.3 M, at least 0.4 M, at least 0.5 M, at least 0.6 M, at least 0.7 M, at least 0.8 M, at least 0.9 M, at least 1 M, at least 2 M or at least 3 M. Preferably, the polyol-based cryoadditives, preferably glycerol 5 %, may be used at a concentration of about 0.6 M to 0.7 M, more preferably at a concentration of 0.68 M.

Alternatively, the polyol-based cryoadditive may be added based on volume percent of the composition comprising pentamers and/or VLP. Suitable volume percent include 3 % (v/v), 4 % (v/v), 5 % (v/v), 6 % (v/v), 7 % (v/v), 8 % (v/v), 9 % (v/v) or 10 % (v/v). Preferentially, the polyol-based cryoadditive is added at 5 % (v/v).

In a preferred embodiment of the invention, the pentamers of step c) and/or the VLP of step d) are subject to purification. The term "purification" in the context of the present invention refers to isolating or separating the VP1, pentamers or VLP from a complex composition. Possible methods include precipitation, preferably centrifugation, filtration, such as ultrafiltration and/or cross flow filtration, preferably cross flow filtration, chromatography, for example a preparative chromatography, preferably size exclusion chromatography or affinity chromatography, and/or dynamic light scattering (DLS). In particular, the VLP can be selected with DLS. Vivaspin® is an exemplary filtration unit which can be used.

The term "chromatography" refers to a method which permits the separation of a mixture of substances by distributing the individual components thereof between a stationary phase and a mobile phase. In particular, chromatography refers to a method of purifying a substance by first binding and enriching the substance of interest to the stationary phase before eluting it in a second step (bind-and-elute mode of chromatography) or by binding impurities to the stationary phase and increasing the purity of the molecule of interest in the flow-through (flow-through mode).

Chromatography can be grouped according to the basis of the interaction of the analyte comprised in the mobile phase with the stationary phase. Preferred types of chromatography according to the invention encompass "reversed phase" chromatography, "ion-exchange" chromatography, "affinity" chromatography, or size exclusion chromatography (SEC). Among ion-exchange chromatography, the purification method can be further separated based on the charge present in the stationary phase into cation-exchange chromatography (CEX), in which the stationary phase has a negative charge, thus retaining positively charged molecules, and anion exchange chromatography (AEX), in which the stationary phase has a positive charge, thus retaining negatively charged molecules.

In particular, chromatography may be used to purify rVLP obtained by the method according to the present invention as an intermediate product. In particular AEX may be used to purify rVLP.

The stationary phase used in AEX can be further described based on the strength of the ionic interaction provided by the exchanging material present in the stationary phase into "strong anion exchangers" and "weak anion exchangers". The expressions "anion exchanger" or "anion exchange matrix" are synonymous and both refer to natural or artificial substances which can bind anions and can exchange these for anions from a surrounding medium. An anion exchanger carries positive ions and exchanges negatively charged counterions.

The VLP of the invention may be further treated with a nuclease and/or be subjected to sterile filtration. A nuclease treatment is preferably done if a nucleic acid, such as an expression vector, is used as cargo. Different nucleases are known to the skilled person and include for example benzonase. Nucleases are used to hydrolyse residual DNA which has not been associated with the VLP. Methods for sterile filtration include, inter alia diafiltration or ultracentrifugation using filters suitable for the removal of impurities. These treatments are especially advantageous for the clinical application of the inventive VLP.

The particle size distribution of a composition comprising VLP according to the invention can be assessed as the "Poly Dispersity Index" (PDI). The PDI indicates the distribution of particle sizes in a composition, and thus describes the uniformity of particles. PDI values can be obtained using different methods, including gel permeation chromatography/size exclusion chromatography, rheology, solution viscosity, membrane osmosis or light scattering.

The PDI preferably is determined by dynamic light scattering (DLS). In DLS, the native distribution is the intensity distribution which indicates how much light is scattered from the various "slices". DLS allows determination of the mean size and the standard deviation from this mean size from the statistics of the distribution. Relative polydispersity can be determined by dividing the standard deviation by the mean. From the relative polydispersity of a distribution the polydispersity index (PDI) can be derived as its square. PDI values obtained by DLS can be grouped into monodispersed (PDI < 0.1) compositions and polydispersed (PDI > 0.1) compositions, whereby also within the polydispersed group smaller values indicate a more uniform distribution within the composition.

According to the invention, PDI values of 0.1 to 0.4 are preferred. More preferably, the PDI value is 0.1 to 0.3 and even more preferably 0.1 to 0.2.

The average diameter of a composition comprising VLP according to the invention may be measured by visual methods, such as microscopy, preferably equipped with software to determine the average diameter, but also by analytic light scattering methods, such as DLS or nanotracking method, such as NTA.

The VLP content within the composition can be measured by, for example, FFF-MALS and/ or DLS. Both methods can distinguish between the right-sized VLP and aggregates, "tinies" (small particles) and other impurities, such as salts, debris or pentamers.

Such a composition comprising VLP according to the invention in particular fulfills requirements usually imposed on a drug delivery system. Obviously, such a composition is homogeneous and has a high purity.

In another aspect, the invention relates to a drug delivery system obtainable by the method according to the invention. Such a drug delivery system has the advantages as stated *supra.* In particular, such a drug delivery system can be used in a method of therapy and/or diagnosis, preferably for the treatment of neurological disorders, i. e. CNS diseases, namely a lysosomal storage disease, in particular MLD.

Hence the invention also relates to a method of treating a disorder, in particular a CNS disease, namely a lysosomal storage disease, in particular MLD, with the drug delivery system according to the invention. The method of treating preferably comprises the step of administering the drug delivery to a subject in need thereof.

The invention also relates to the use of the drug delivery system for the manufacture of a medicament for the treatment of neurological disorders, i. e. CNS diseases, namely a lysosomal storage disease, in particular MLD. The method of treatment preferably does not comprise a step of increasing the permeability of the BBB of the subject to be treated. The drug delivery system of the invention, preferably, is administered to a patient who has not received any chemical or physical treatment for impairing or disrupting the BBB.

In one embodiment, the VLP according to the invention cross the blood brain barrier (BBB). Therefore, in one embodiment of the invention, the drug delivery system may be used to deliver the lysosomal enzyme or the expression vector encoding a lysosomal enzyme across the BBB. According to the invention, a drug delivery system and/or a VLP and/or a lysosomal enzyme and/or an expression vector encoding a lysosomal enzyme may cross the BBB.

Importantly, the crossing of the BBB by the drug delivery system enables the drug delivery system to exhibit its function of targeting specific cell populations within the brain, i.e. deliver a cargo to targeted cells. In the context of the invention said drug delivery system comprises a delivery to and/or into the targeted cells.

In a preferred embodiment, the VLP of the invention and/or its cargo, after administration to the subject to be treated, in particular a human, can be detected in the CNS in less than 10 days, preferably in less than 5 days, more preferably in less than 3 days after administration. The drug delivery system preferably is administered to the subject intravenously. This is particularly advantageous when using the VLP as a reliable drug delivery system.

Preferably, the method does not require a loss of integrity or increased permeability of the BBB.

According to the invention, it is not required to impair the permeability of the BBB prior or while administering the drug delivery system. Thus, the BBB is preferably physiologically intact which means that the integrity is not decreased and/or the permeability not increased compared with the healthy, native state. The VLP of the invention preferably cross the physiologically intact BBB.

The composition comprising the drug delivery system preferably does not require an additive that may disrupt the integrity of the BBB. Hence, in a most preferred embodiment of the invention the drug delivery system is free of any additive that can impact the permeability of the BBB.

### MATERIALS AND METHODS

### VLP manufacturing

Virus-like particles (VLP) were manufactured by protein expression using a Sf9 insect cell line derived from the fall armyworm (Spodoptera frugiperda) (Thermo Fischer scientific). VLP were produced by infecting the cells with recombinant Baculovirus containing a John Cunningham virus VP1-protein expression cassette. The recombinant Baculovirus was prepared by using the Bac-to-Bac® Baculovirus expression system (Thermo Fischer Scientific). VLP were produced at pH 6.3 after 7 to 10 days in a 3.4 L bioreactor (INFORS HT Minifors). Air flow and temperature (26°C) were controlled over the time. To remove cells and cell debris suspension was centrifuged at 4°C, 5.000 g and the supernatant containing VLP was harvested.

After that VLP were concentrated using two different concentration methods: precipitation with 7.5 % polyethylenglycol (PEG) or cross flow with an ÄKTAcross flow™ system (GE Healthcare). For PEG precipitation the clarified supernatant was mixed with PEG to achieve 7.5% (v/v) and incubated for 2 h at 4°C, after that the precipitate was separated by centrifugation at 4°C, 10.000 g and suspended in 50 mM NaCl, 10 mM Tris-HCl, pH 7.5. Cross flow was performed with an ÄKTAcross flow™ system equipped with a 300 kDa cut-off membrane (Hydrosart® 300kDa ECO, Sartorius). The flow ultrafiltration was carried out with a constant pressure of 1.5 bar and a factor of 8 (1 L supernatant against 8 L buffer).

VLP were further dissociated to pentamers by using 5mM DTT and 10 mM EDTA for 70 min at room temperature and the pentamers purified by anion exchange chromatography (AEX) using HiScale CaptoQ column (GE Healthcare) with a NaCl step gradient from 150 mM to 1M NaCl. Pentamers were eluted with a 250 mM NaCl step. After elution the pentamers were processed as follows:
Immediately placed into dialysis cassettes with 20 kDa cut-off (Slide-A-Lyzer™ G2 Dialysis Device, Thermo Scientific) and reassembled by two-step-reassembly by dialysis (two-step dialysis). First, pentamers were dialyzed against 2 M ammonium sulfate buffer ("AS", 10 mM Tris-HCl, 150 mM NaCl, 2 M (NH4)2SO4, pH 7.5) for 24 h and then transferred for the next 24 h into 10 mM Tris-HCl, 150 mM NaCl, 2 mM CaCl2, pH 7.5 (standard reassociation buffer, "ST"). To separate not reassembled material and aggregates from the VLP, the composition comprising the VLP was purified by size exclusion chromatography (SEC) using HiPrep™ Sephacryl® S-500 HR column (GE Healthcare) under control of polydispersity index (PDI) of fractions in dynamic light scattering (DLS) using Zetasizer ZS Nano (Malvern Inc.). VLP fraction with targeted size were selected and pooled, and then concentrated in Vivaspin® concentrators (Sartorius) with 5 kDa cut-off membrane, if applicable followed by storage at -80°C.

### Detection of VLP in oligodendrocytes, neurons and microglia

10 µm thin mouse brain slides were re-hydrated in xylol, subsequent ethanol concentrations from 100% to 70% and then with water. After that the endogen peroxidase was inactivated with 0.5% H2O2 and unspecific binding sites were blocked with 3% bovine serum albumin solution. VP1 protein was detected with VP1 antibody (diluted 1:500 in milk) in combination with fluorescence-conjugated secondary antibody. Brain cells were stained with cell-specific markers:
Olig2 -oligodendrocytes (diluted 1:200 in milk)
NeuN - neurons (diluted 1:200 in milk)
IbaI - microglia cells (diluted 1:200 in milk).
Cell nuclei were stained with Hoechst33342 reagent (2 µg/ml). Slides were visualized with a fluorescent microscope (Nikon).

### Artificial blood-brain-barrier (BBB) model for verifying BBB permeation

BBB permeation of VLP was assessed by using monoculture in a two-compartment artificial BBB model by direct quantification of permeated fluorescently labeled-material: 4 kDa FITC-Dextran, 70 kDa TRITC-Dextran, FITC-labeled pNL1.1 plasmid (construct) or FITC-labeled pNL1.1 plasmid packed into VLP (packaging).

For that purpose, 24-well permeable supports (insert, 1µm, ThinCert, Greiner Bio-One) were transferred to a 24-well plate (Greiner Bio-One) filled with 900 µl medium (DMEM/F-12, HEPES, Thermo Fisher Scientific). Subsequently, 7.5*10⁴ BBB cells (HBEC-5i, cerebral microvascular endothelium) were seeded into the inserts with 200 µl of the same medium. Cells were incubated for 96 h to 120 h with exchanging the media in the wells every second day.

The prepared inserts were carefully transferred to a fresh 24-well plate filled with 900 µl of phenol-free medium (DMEM/F-12, HEPES, no phenol red, Thermo Fisher Scientific) per well. Prior to adding the sample to the insert, the corresponding amount of media was removed, for keeping a constant amount of 200 µl medium in each insert. After 120 min of incubation at 37°C in an incubator, 3x 100 µl of each well were transferred to a black 96-well plate and the fluorescence of the sample was determined using a plate reader (Tecan Infinite M200, Tecan).

Wheat germ agglutinin (WGA) staining was performed by staining the cells' membrane with antibody against WGA conjugated with Alexa488 for 15+ min at room temperature. To confirm BBB formation, the BBB was disrupted by the addition of EDTA to the insert. The inserts were transferred to a 24-well plate containing fresh phenol-free media. EDTA (cEnd = 5 mM, approx. 5 µl) was added to the inserts and incubated for 90 min at 37°C. Afterwards, fluorescence was determined as described above. Permeation was calculated by normalizing the fluorescence signal of the inserts harboring BBB cells to the appropriate inserts without BBB cells. In the same manner, the integrity of the BBB was verified after the addition of EDTA.

### Cloning of an expression vector encoding hASA

Expression vectors:
1) pFAR-CMV-ASA (3.5 kb)
2) pFAR-CAG-ASA (4.6 kb)
3) pNL-BB-ASA (4.9 kb)
4) pSF-CAG-ASA cut (3.1 kb + 3.7 kb*)

Construct 1 and construct 2 were generated from the pFAR4 plasmid backbone (as disclosed in SEQ ID NO: 21 of US 8,440,455 B2 and herein disclosed as SEQ ID NO: 7), the CMV and CAG promoter, respectively, and the human ASA gene (SEQ ID NO: 2).

For generating construct 3 (4.9kb), the hASA gene (SEQ ID NO: 2) was inserted into a pNL1.1 plasmid backbone (Promega) using Gibson assembly. The construct comprises a CMV promoter.

For generating construct 4 (3.1kb + 3.7kb), the hASA gene (SEQ ID NO: 2) was inserted into a pSF-CAG plasmid backbone (Oxford genetics) and then was linearized by restriction digestion of a hASA expression vector with help of two restriction enzymes.

### Packaging of VLP with the expression vector encoding hASA

For packaging hASA expression vectors into VLP, pre-reassembled VLPs were taken from -80°C and thawed using a thermal shaker (23°C, 350 rpm). Subsequently, VLPs were dissociation by incubating the samples for 15 min at 23°C and 450 rpm in the presence of dissociation buffer (20 mM Tris-HCl, 150 mM NaCl, 5 mM DTT, and 10 mM EDTA). Dissociated VLP were reassembled in the presence of hASA expression vectors. In short, the dissociated VLP were mixed thoroughly with the hASA expression vector in appropriate concentrations (packaging ratio VLP to expression construct 1: 0.2) followed by dialyzing the mixture against standard reassociation buffer (10 mM Tris-HCl, 150 mM NaCl, 2 mM CaCI2, pH7.5) using a 20 kDa MWCO dialysis chamber (Slide-A-Lyzer™ MINI Dialysis Device, Thermo Scientific). Samples were incubated for 16 to 18 h. Samples were transferred from the dialysis chamber to a fresh reaction cup and the unpacked nucleic acids, i. e. expression vectors, were digested by incubation with 40 u Benzonase® Nuclease (Merck) per 25 µg VLP and 2.5 mM MgCl2 at 37°C for 1 h. Samples were filtrated (Corning® Costar® Spin-X® centrifuge tube filters; pore size 0.22 µm) and frozen in liquid N2. Afterwards, VLP were analyzed and/or stored at -80°C.

### VLP characterization

For verifying sample stability, inflection temperatures for each sample were assessed by nDSF using a Tycho NT.6 (Nanotemper).

To analyze sample composition, asymmetric flow field flow fractionation (AF4, Wyatt Inc.) was performed. Samples were analyzed by using multiangle light scattering (MALS), dynamic light scattering (DLS) and UV detector.

Transmission electron microscopy (TEM) analyses were carried out for directly visualizing the sample at different experimental steps with help of a Zeiss EM900 electron microscope, operating at a voltage at 80 kV. For this approach, samples were stained on carbon-coated copper grids (Plano GmbH) using 2 % uranyl acetate (Sigma Aldrich) beforehand.

### hASA expression in cells: RNA Isolation, cDNA Synthesis and qPCR

After incubating the cells for 72 h with the samples (i. e. VLP associated with a lysosomal enzyme or an expression vector encoding a lysosomal enzyme), glioblastoma cells were harvested using trypsin, pelleted, and washed using PBS.

The RNA was isolated from the cell pellet using an RNA Isolation Kit (Macherey-Nagel). After Isolation, RNA concentration was determined using NanoDrop (Thermo Scientific).

For cDNA synthesis 250ng RNA were used. Afterwards, cDNA samples were diluted 1:5 in ddH₂O and samples were stored at -20°C until use.

qPCR was performed in a Lightcycler (CFX96 Touch, BioRad) using EvaGreen Dye and the appropriate primers for hASA in an 1:10 dilution.

### Injection of mice with VLPlhASA

Animals: BALB/cJ males, provided from Janvier Labs, 10 weeks of age at begin of the study.

**Groups:**

| Group | Time point 1 (72h) | Time point 2 (120h) |
|---|---|---|
| Group 1 - vehicle | 2 mice (1-2) | 2 mice (3-4) |
| Group 2 - pCAG plasmid | 2 mice (9-10) | 2 mice (11-12) |
| Group 3 - EPCs + pCAG plasmid | 5 mice (23-27) | 5 mice (28-32) |

The animals were injected once i.v. with 180 µl of the test compounds or vehicle. Half of the mice in each treatment group were terminated 72 h post injection, the other half was terminated 120 h post injection.

Upon termination the animals were perfused with Tris-buffered saline and the following organs sampled: brain, kidney, liver, lung, heart. Tissue samples were shock-frozen in liquid N2 and stored at -80°C.

### mRNA analysis with qPCR in liver and brain

A part of tissue was placed in 500 µl TBS+0.5% Triton-X100 with RNAse and homogenized with sonication 3 cycles 20 sec each. Samples were centrifuged 10 min at 11.000 rpm. The RNA was isolated from the tissue using an RNA Isolation Kit (Macherey-Nagel). After Isolation, RNA concentration was determined using NanoDrop (Thermo Scientific).

For cDNA synthesis 250ng RNA were used. Afterwards, cDNA samples were diluted 1:5 in ddH₂O and samples were stored at -20°C until use.

qPCR was performed in a Lightcycler (CFX96 Touch, BioRad) using EvaGreen Dye and the appropriate primers for hASA in an 1:10 dilution.

### Western Blot

A part of the tissue was placed in 500 µl TBS+ 0.5% Triton-X100 and homogenized with sonication 3 cycles 20 sec each. Samples were centrifuged 10 min at 11.000 rpm and protein concentration was measured with BCA assay. 50 µg of each sample were placed into a gel (4-20% gradient SDS-PAGE) and standard electrophoresis and blotting protocol were performed. Antibody staining (hASA antibody, 1:500 diluted in milk) was prepared after washing with buffer and blocking in 5% milk overnight at 4°C. Secondary antibody staining was performed with HRP-conjugated goat anti-mouse antibody. After staining the membrane was washed with buffer and developed. After that the membrane was washed with buffer, stripped with stripping buffer and stained with actin (1-19) HRP-conjugated antibody (1:1000 dilution in milk) for the actin control staining.

### EXAMPLES

### 1. Colocalization analysis reveals presence of VP1 protein in oligodendrocytes

A co-localization experiment was performed in order to identify the respective target cells of the VLP. For this purpose, the VP1 protein was co-localized with the marker Olig 2, which is specifically located on oligodendrocytes. As shown in the lower left panel of Figure 1, by using the Olig2 marker, several irregular spots could be detected in the brain slice, which in every case are also stained by the nucleus stain DAPI (upper left panel of Figure 1) and therefore indicative of oligodendrocytes. Each of these oligodendrocytes is also positive for VP1 protein as shown in the lower right panel of Figure 1. The cells which are positive for Olig2 and VP1 are marked with a white arrow. Hence, the VP1 protein is localized in CNS oligodendrocytes. As a result the VLP of the present invention are especially suited for a therapeutic treatment of diseases associated with oligodendrocytes, for example MLD.

### 2. Colocalization analysis reveals presence of VP1 protein and luciferase in neurons

In another co-localization experiment the VP1 protein was co-localized with the marker NeuN, which is specifically located on neurons. As shown in the lower left panel of Figure 2, by using the NeuN marker several irregular spots could be detected in the brain slice, which in every case are also stained by the nucleus stain DAPI (upper left panel of Figure 2) and therefore indicative of neurons. Each of these neurons is also positive for VP1 protein as shown in the lower right panel of Figure 2. The cells which are positive for NeuN and VP1 are marked with a white arrow. Hence, the VP1 protein is localized in CNS neurons.

### 3. Colocalization analysis reveals presence of VP1 protein and luciferase in microglia cells

In another co-localization experiment the VP1 protein was co-localized with the marker Iba1, which is specifically located on microglia cells. As shown in the lower left panel of Figure 3, by using the Iba1 marker several irregular spots could be detected in the brain slice, which in every case are also stained by the nucleus stain DAPI (upper left panel of Figure 3) and therefore indicative of microglia cells. Each of these microglia cells is also positive for VP1 protein as shown in the lower right panel of Figure 3. The cells which are positive for Iba1 and VP1 are marked with a white arrow. Hence, the VP1 protein is localized in CNS microglia cells.

### 4. VLP associated with a pNL plasmid show a good permeation across the BBB in an in vitro BBB model

Figure 4A and B show an *in vitro* BBB permeation model. Figure 4A shows DAPI staining (left) of cell nuclei and WGA staining (right) of the cell membranes of BBB-cells. The cell layer is intact representing an intact BBB.

Figure 4B shows the permeation of fluorescence dyes (FITC, TRITC), a fluorescent dye coupled to a plasmid (FITC-pNL) and a VLP associated with a pNL plasmid across the BBB model. Permeation in % in relation to a permeation without cells is shown. Left four columns are samples without EDTA, right four columns samples with EDTA. As can be seen, while all four samples crossed the artificial BBB to some extent, the sample "Packaging" which represents the VLP associated with a pNL plasmid showed the highest permeation. Disruption of the BBB by EDTA opened the BBB for all four samples.

### 5. Significant increase of ASA mRNA levels in the brain of healthy mice

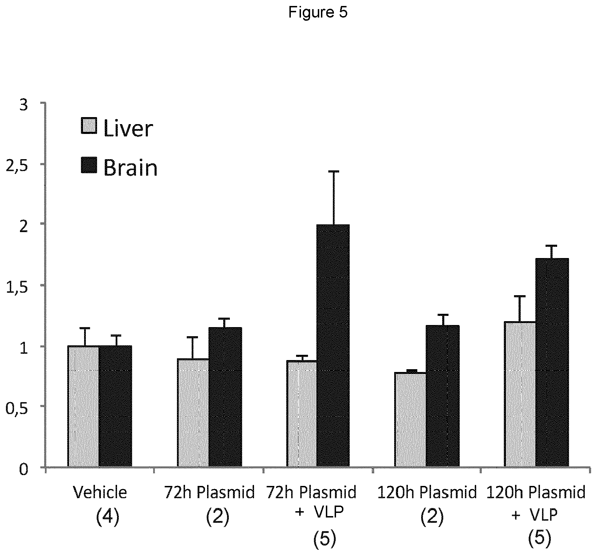

An mRNA analysis in liver and brain of healthy mice injected with VLP associated with an expression vector encoding ASA revealed a significant increase of ASA mRNA in the brain after 72 and 120 h (Figure 5). Injection of the expression vector (plasmid) without VLP did not lead to an increased expression. Hence, expression is specifically found in the brain.

### 6. Single mouse analyses of ASA mRNA in the brain

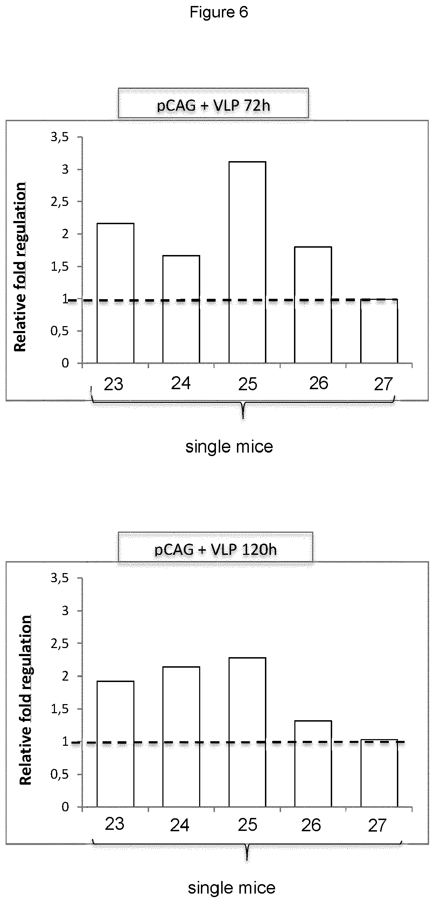

Single mouse analyses after 72 h (top) and 120 h (bottom) after injection of VLP associated with an expression vector encoding ASA (Figure 6). Numbers denote single mice. In almost all mice of these analyses an increased ASA expression was observed.

### 7. Single mouse analyses of ASA protein in the brain

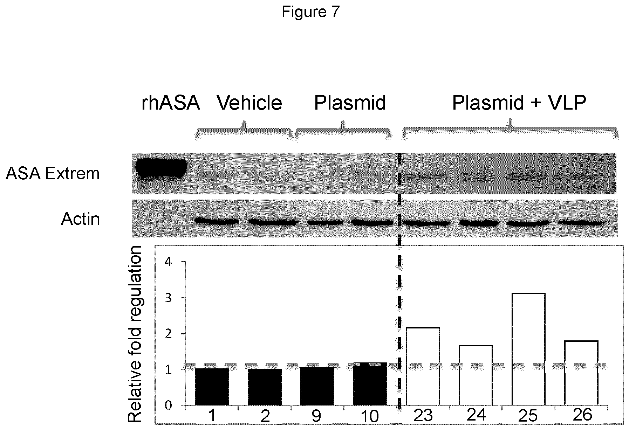

Single mouse analysis of ASA protein (Western blot, top) in the brain of healthy mice after injection of VLP associated with an expression vector encoding ASA and corresponding qPCR analysis (bottom) is shown in Figure 7. Vehicle (buffer), plasmid and plasmid + VLP are compared. It is evident that association of the VLP with a plasmid lead to an increased mRNA and protein expression of ASA in the brain.

### 8. ASA expression with different hASA expression constructs

Figure 8 shows the expression of human ASA in glioblastoma cells by qPCR. The values were normalized to respective plasmid controls.

The expression vectors in Figure 8A are:
Construct 1: pFAR-CMV-ASA (3.5 kb)
Construct 2: pFAR-CAG-ASA (4.6 kb)
Construct 3: pNL-BB-ASA (4.9 kb)
Construct 4: pSF-CAG-ASA cut (3.1 kb + 3.7 kb*)

The packaging ratio VLP: plasmid was 1:0.2. All four expression vectors lead to ASA expression.

In Figure 8B the Construct 4 of A (pSF-CAG-ASA cut (3.1 kb + 3.7 kb*)) was used at two different packaging ratios (high concentration [VLP: plasmid of 1:0.5] and low concentration [VLP: plasmid of 1:0.2]). It can be seen that the low concentration [packaging ratio VLP: plasmid of 1:0.2] leads to a higher ASA expression.

### 9. VLP analyses after association with different hASA expression constructs: nDSF and AF4 analyses

Figure 9A shows by means of nDSF analyses that all four samples (VLP associated with the expression vectors of Figure 8A) are stable. The AF4 analyses (Figure 9B) further show that the fraction of VLP is superior over the fractions of tinies, aggregates and residuals in that most of the detected particles are VLP, i. e. the VLP are very homogeneous.

### 10. TEM images of VLP associated with expression vectors

Figure 10 shows a comparison of VLP unpacked (without associated expression vector) (left) and VLP associated with four different expression vectors (right; the same expression vectors as in Figure 8A). As can be seen, all samples show VLP which are visually the same as the unpacked VLP.

### MATERIALS AND METHODS FOR FURTHER EXAMPLES

### VLP manufacturing

Virus-like particles (VLP) were manufactured by protein expression using a Sf9 insect cell line derived from the fall armyworm (*Spodoptera frugiperda*) (Thermo Fischer scientific). VLP were produced by infecting the cells with recombinant Baculovirus containing a John Cunningham virus VP1-protein expression cassette. The recombinant Baculovirus was prepared by using the Bac-to-Bac® Baculovirus expression system (Thermo Fischer Scientific). VLP were produced at pH 6.3 after 7 to 10 days in a 3.4 L bioreactor (INFORS HT Minifors). Air flow and temperature (26°C) were controlled over the time. To remove cells and cell debris suspension was centrifuged at 4°C, 5.000 g and the supernatant containing VLP was harvested.

After that VLP were concentrated using two different concentration methods: precipitation with 7.5 % polyethylenglycol (PEG) or cross flow with an ÄKTAcross flow™ system (GE Healthcare). For PEG precipitation the clarified supernatant was mixed with PEG to achieve 7.5% (v/v) and incubated for 2 h at 4°C, after that the precipitate was separated by centrifugation at 4°C, 10.000 g and suspended in 50 mM NaCl, 10 mM Tris-HCl, pH 7.5. Cross flow was performed with an ÄKTAcross flow™ system equipped with a 300 kDa cut-off membrane (Hydrosart® 300kDa ECO, Sartorius). The flow ultrafiltration was carried out with a constant pressure of 1.5 bar and a factor of 8 (1 L supernatant against 8 L buffer).

VLP were further dissociated to pentamers by using 5mM DTT and 10 mM EDTA for 70 min at room temperature and the pentamers purified by anion exchange chromatography (AEX) using HiScale CaptoQ column (GE Healthcare) with a NaCl step gradient from 150 mM to 1M NaCl. Pentamers were eluted with a 250 mM NaCl step. After elution the pentamers were processed as follows:
a) Mixed with cargo and reassembled (see "Packaging of fresh or stored pentamers with cargo");
b) Frozen at -80°C with 5% glycerol for storage of pentamers (which can subsequently be mixed with cargo and reassembled, see "Packaging of fresh or stored pentamers with cargo") or
c) Immediately placed into dialysis cassettes with 20 kDa cut-off (Slide-A-Lyzer™ G2 Dialysis Device, Thermo Scientific) and reassembled by two-step-reassembly by dialysis (two-step dialysis). First, pentamers were dialyzed against 2 M ammonium sulfate buffer ("AS", 10 mM Tris-HCl, 150 mM NaCl, 2 M (NH₄)₂SO₄, pH 7.5) for 24 h and then transferred for the next 24 h into 10 mM Tris-HCl, 150 mM NaCl, 2 mM CaCl₂, pH 7.5 (standard reassociation buffer, "ST") (AS>ST). Alternatively, the two-step dialysis was performed with ST buffer in both steps (ST>ST). In both cases, to separate not reassembled material and aggregates from the VLP, the composition comprising the VLP was purified by size exclusion chromatography (SEC) using HiPrep™ Sephacryl® S-500 HR column (GE Healthcare) under control of polydispersity index (PDI) of fractions in dynamic light scattering (DLS) using Zetasizer ZS Nano (Malvern Inc.). VLP fraction with targeted size were selected and pooled, and then concentrated in Vivaspin® concentrators (Sartorius) with 5 kDa cut-off membrane, if applicable followed by storage at -80°C.

### Packaging of reassembled VLP with cargo

For VLP packaging of reassembled VLP, stored VLP of step c) above were taken from -80°C and thawed using a thermal shaker (23°C, 350 rpm). Subsequently, dissociation of thawed VLP samples was performed by incubating the samples for 15 min at 23°C and 45 rpm in the presence of dissociation buffer (20 mM Tris-HCl, 150 mM NaCl, 5 mM DTT, and 10 mM EDTA). Dissociated VLP were reassembled in the presence of cargo (e.g., nucleic acid). In short, the dissociated VLP were mixed thoroughly with the cargo in appropriate concentrations followed by dialyzing the mixture against standard reassociation buffer (10 mM Tris-HCl, 150 mM NaCl, 2 mM CaCl₂, pH7.5) using a 20 kDa MWCO dialysis chamber (Slide-A-Lyzer™ MINI Dialysis Device, Thermo Scientific). After 4 to 6 h, dialysis buffer was replaced by fresh dialysis buffer and samples were incubated for additional 16 to 18 h. In case of using nucleic acids as cargo, unpacked nucleic acids were digested by incubation with 40 u Benzonase® Nuclease (Merck) per 25 µg VLP and 2.5 mM MgCl₂ at 37°C for 1 h. Samples were filtrated (Corning® Costar® Spin-X® centrifuge tube filters; pore size 0.22 µm) before analysis. VLP were directly analyzed afterwards and/or stored at -80°C.

### Packaging of fresh or stored pentamers with cargo

For pentamer packaging, fresh or at -80°C stored pentamers were directly mixed and reassembled with cargo or thawed using a thermal shaker (23°C, 350 rpm), and then reassembled in the presence of cargo (e.g., nucleic acid). In short, the pentamers were mixed thoroughly with the cargo in appropriate concentrations followed by dialyzing the mixture against standard reassociation buffer (10 mM Tris-HCl, 150 mM NaCl, 2 mM CaCl₂, pH7.5) using a 20 kDa MWCO dialysis chamber (Slide-A-Lyzer™ MINI Dialysis Device, Thermo Scientific). In case of using nucleic acids as cargo, unpacked nucleic acids were digested by incubation with 40 u Benzonase® Nuclease (Merck) per 25 µg reassembled VLP and 2.5 mM MgCl₂ at 37°C for 1 h. Reassembled pentamers were directly analyzed afterwards.

### Functionality testing of VLP / Luciferase Assay

In case of VLP loaded with nucleic acids (manufactured from fresh or stored pentamers; or from reassembled and purified VLP) luciferase NanoLuc® plasmid has been used as cargo. Glioblastoma cells were seeded 24 h in advance with a density of 3*104 in 900 µl DMEM medium, high glucose, GutaMAX™ (Thermo Fisher) supplemented with 10 % FCS (Thermo Fisher) and 1 % Pen-Strep (PAN-Biotech). Prior to adding the sample the corresponding amount of media was removed for keeping a constant amount of medium in each well. Samples were added to the cells and incubated for 48 h at 37°C and 5 % CO₂. After that cell medium was eliminated and cells were washed with PBS. Luciferase activity was determined according to the manufacturer's instructions (NanoGlo® Luciferase Assay (Promega)). Luminescence was measured in white 96-well plates (Greiner bio-one) in Glomax® Multi detection system (Promega).

### VLP characterization

For verifying sample dissociation, reassembly and stability, dynamic light scattering (DLS) and polydispersity indices (PDI) of the samples were measured using a Zetasizer Nano ZS (Malvern.). The VLP sizes and PDI were documented with Zetasizer Software (version 7.11, Malvern). Additionally, inflection temperatures for each sample were assessed by nDSF using a Tycho NT.6 (Nanotemper).

To analyze sample composition, asymmetric flow field flow fractionation (AF4, Wyatt Inc.) was performed. Samples were analyze by using multiangle light scattering (MALS), dynamic light scattering (DLS) and UV detector.

Transmission electron microscopy (TEM) analyses were carried out for directly visualizing the sample at different experimental steps with help of a Zeiss EM900 electron microscope, operating at a voltage at 80 kV. For this approach, samples were stained on carbon-coated copper grids (Plano GmbH) using 2 % uranyl acetate (Sigma Aldrich) beforehand.

### Artificial blood-brain-barrier (BBB) model for verifying BBB permeation

BBB permeation of VLP was assessed by either using monoculture (direct quantification of permeated fluorescently labeled-material) or co-culture (protein expression caused by permeated material in target cells) in a two-compartment artificial BBB model.

In both cases, 24-well permeable supports (insert, 0.4µm, Corning Costar) were coated for 90 min at 37°C with a mixture of 1:20 Fibronectin (BioReagent) and 1:75 Collagen Type I (Merck Millipore)in phosphate buffered-saline (PBS) in advance. After carefully removing the excess coating, coated inserts were transferred to a fresh 24-well plate (Greiner Bio-One) filled with 900 µl medium (EndoGRO, SCME004, Merck Millipore). 7.5*10⁴ BBB cells (HBEC-5i, cerebral microvascular endothelium) were seeded into the inserts with 200 µl medium. Cells were incubated for 96 h to 120 h with exchanging the media in the wells every second day.

### Monoculture experiments (permeation of fluorescently labeled cargo)

The prepared inserts were carefully transferred to a fresh 24-well plate filled with 900 µl of phenol-free medium (DMEM/F-12, HEPES, no phenol red, Thermo Fisher Scientific) per well. Prior to adding the sample to the insert, the corresponding amount of media was removed, for keeping a constant amount of 200 µl medium in each insert. After 120 min of incubation at 37°C in an incubator, 3x 100 µl of each well were transferred to a black 96-well plate and the fluorescence of the sample was determined using a plate reader (Tecan Infinite M200, Tecan).

To confirm BBB formation, the BBB was disrupted by the addition of EDTA to the insert. The inserts were transferred to a 24-well plate containing fresh phenol-free media. EDTA (c_{End} = 5 mM, approx. 5 µl) was added to the inserts and incubated for 90 min at 37°C. Afterwards, fluorescence was determined as described above.

Permeation was calculated by normalizing the fluorescence signal of the inserts harboring BBB cells to the appropriate inserts without BBB cells. In the same manner, the integrity of the BBB was verified after the addition of EDTA.

### Co-culture experiments (activity of permeated material)

Inserts were transferred to a fresh 24-well plate, containing target cells (glioblastoma cells), that were seeded 24 h in advance with a density of 3*10⁴ in 900 µl DMEM, high glucose, GutaMAX™ (Thermo Fisher) supplemented with 10 % FCS (Thermo Fisher) and 1 % Pen-Strep (PAN-Biotech). Prior to adding the sample (VLP packed with NanoLuc® plasmid) to the insert, the corresponding amount of media was removed for keeping a constant amount of 200 µl medium in each insert. After 24 h, the insert was removed from the 24-well plate and washed using PBS. Accordingly, the membrane (harboring the BBB cells) was cut out using a scalpel and transferred to a 1.5 ml reaction vial. Luciferase activity was determined according to the manufacturer's instructions (NanoGlo® Luciferase Assay (Promega)).

The wells (containing the target cells) were incubated for another 24 h before the luciferase assay was performed according to the manufacturer's instructions (NanoGlo® Luciferase Assay (Promega)).

### Storage with cryoprotection

Each cryoadditive glycerol (Carl Roth), sucrose (Carl Roth), ammonium sulfate (Carl Roth), DMSO (Carl Roth) and betaine (Merck) was added to VLP resulting in a final additive concentration of 1 M, except for glycerol with 5 % (v/v) corresponding to 0.68 M. Additionally, one aliquot did not contain an additive substance (w/o). The samples were splitted and frozen by applying two freezing speeds: One half of each sample was frozen with a cooling rate of approx. -1°C/min (slow freezing), while the second half was frozen fast by dipping the containers into liquid nitrogen (fast freezing). All samples were stored at -80°C with a storage period of 1 to 10 days. After thawing at 23°C and shaking at 350 rpm, the samples were dialyzed against 10 mM Tris-HCl, 150 mM NaCl, 2 mM CaCl₂, pH 7.5 at room temperature for 2 h to reduce additive concentration and their impact on analysis results. Particle diameter and PDI were determined by a Zetasizer Nanoseries (Malvern). Inflection temperatures were measured using a Tycho NT.6 (Nanotemper). VLP functionality after storage was proved with help of NanoLuc® plasmid packaging and subsequent NanoGlo® Luciferase assay (Promega).

### FURTHER EXAMPLES

### Example 11: Functional analyses

The cargo delivering efficacy of VLP, which were prepared using different methods, was compared (Fig. 11). To this end, VLP were generated that underwent two rounds of disassembly and subsequent reassembly or one round of disassembly and subsequent reassembly (in both methods, last reassembly in the presence of a luciferase plasmid). Remarkably, Luciferase activity of VLP that underwent two rounds of disassembly and subsequent reassembly was significantly increased compared to VLP that underwent one round of disassembly and subsequent reassembly and compared with the "plasmid only" and "cells only" controls.

Next, the ability of VLP prepared with two rounds of disassembly and subsequent reassembly to penetrate the blood-brain-barrier (BBB) was analyzed (Fig. 12). Figure 12A shows the setup of an artificial blood-brain-barrier (BBB) model for testing BBB permeation by VLP or plasmid (circles) in a monoculture setup. Human brain endothelial cells (BBB cells, HBEC-5i) were plated on the permeable support. Figure 12B shows the permeability of the VLP that underwent two rounds of disassembly and subsequent reassembly (second reassembly in the presence of a fluorescently labeled plasmid) compared with a "plasmid only" control. The black columns represent experiments with the intact BBB. The white columns represent experiments with an artificially disrupted BBB (by the addition of EDTA). Strikingly, VLP prepared with two rounds of disassembly and subsequent reassembly were capable to cross the BBB in significantly larger ratios than naked plasmid. Disruption of the integrity of the BBB layer destroyed this difference, showing the selective, efficient penetration capacity of VLP prepared with two rounds of disassembly and subsequent reassembly.

To assess the delivering efficacy of VLP generated with two rounds of disassembly and subsequent reassembly into target cells a co-culture BBB model was used (Fig. 13). Figure 13A shows the setup of an artificial BBB model for testing BBB permeation by VLP or plasmid (circles) and subsequent protein expression caused by permeated material in target cells in a co-culture setup. Figure 13B shows the Luciferase activity that was measured in the BBB cells present in the insert (in HBEC-5i cells) and Luciferase activity in the target cells after permeation. An artificial BBB ("Coating + BBB cells") and a coating without BBB cells ("Coating only") were tested. Luciferase actitivity of the cells of the inserts was only measured when there were cells present (thus, the "Coating only" samples did not show any luciferase activity as expected) and when the luciferase plasmid was packed into VLP that were prepared with two rounds of disassembly and subsequent reassembly (compared with plasmid only). Luciferase activity of the target cells was also only measured in the samples with the VLP prepared with two rounds of disassembly and subsequent reassembly (compared with plasmid only) irrespective if the BBB cell layer was present or only the coating.

Thus, VLP prepared with two rounds of disassembly and subsequent reassembly are capable of efficiently passing through the BBB while retaining a high infectivity and cargo delivering capacity. Sample (packed) means VLP that underwent two rounds of disassembly and subsequent reassembly (second reassembly in the presence of a luciferase plasmid).

### Example 12: Storing pentamers vs. storing VLP

To investigate the homogeneity and uniformity of compositions comprising loaded VLP that had undergone different storing procedures, transmission electron microscopy (TEM) was performed. VLP that were generated with one round of disassembly and subsequent reassembly (pentamers frozen, left) were compared with VLP that were generated with two rounds of disassembly and subsequent reassembly (VLP frozen, right) (Fig. 14). The VLP generated in the latter case showed a significantly more uniform distribution as they lacked any visible defective viral particle structures when compared to the VLP wherein the pentamers were frozen during manufacturing.

To further investigate the homogeneity of loaded VLP generated as in Figure 14, FFF-MALS analyses were performed (Fig. 15). Both samples were compared with freshly prepared VLP (without cargo, thus without disassembly/reassembly), serving as reference. Strikingly, compared with the reference (Fig. 15 C), VLP generated after two rounds of disassembly and subsequent reassembly with intermediate freezing of VLP (Fig. 15 A) significantly reduced the amount of VLP aggregates present in the composition. In addition, the distribution of VLP was even more uniform. The size of the VLP and the presence of "Tinies" and "Salts, debris, pentamers" was comparable.

Remarkably, when VLP were generated after one round of disassembly and subsequent reassembly (pentamers frozen) very few structurally intact VLP could be identified (Fig. 15 B). Therefore, the method with two rounds of disassembly and subsequent reassembly allows the intermediate storage of VLP as reassembled VLP and the generation of a highly uniform population of loaded VLP with almost no aggregates. Percentages are given excluding the aggregates.

To further characterize the stability of VLP that were generated after two rounds of disassembly and subsequent reassembly with intermediate freezing of VLP ("Sample (packed)", solid line) compared with freshly prepared VLP (without cargo, thus without disassembly/reassembly, "Sample (wt)", dotted line) nDSF analyses were carried out in order to reveal melting temperatures (inflection temperatures) (Fig. 16). Remarkably, the melting curves of both VLP samples were hardly distinguishable showing that VLP prepared with two rounds of disassembly and subsequent reassembly are equally stable as freshly prepared VLP.

### Example 13: Inducing aggregation before reassembly

Next, the effect of inducing aggregation during reassembly of pentamers into VLP was measured. Homogeneity was analyzed by DLS (Figs. 17 and 18). Figure 17 shows the PDI of VLP obtained after dialysis with induction of aggregation (white column) and without induction of aggregation (black column). VLP reassembled by first inducing aggregation showed a strongly reduced PDI compared with the VLP reassembled without induction of aggregation. Therefore, the induction of aggregation during reassembly led to a much more homogenous size distribution. Both samples were taken before freezing (thus, they underwent one round of disassembly and reassembly).

Figure 18 shows the average size distribution determined by DLS of the samples prepared as in Figure 17 (Fig. 18, left) and samples that had undergone the same reassembly conditions but a subsequent freezing step (Fig. 18, right). Both samples that had been reassembled with induction of aggregation (solid lines) show a very uniform size distribution, while the samples that did not undergo the aggregation step (dotted lines) show at least two VLP populations. Even more, the freezing step was obviously harmful for the latter sample as the composition comprising the VLP that had been reassembled without induction of aggregation had become considerably more heterogeneous after freezing. However, the VLP that had been reassembled with induction of aggregation could be frozen without any impact on the homogeneity of the sample.

### Example 14: Effect of cryoadditives

To further scrutinize the effect of sample storage on VLP, different cryoadditives were added to compositions comprising VLP that were frozen after one round of disassembly and subsequent reassembly and two dialysis steps including AS buffer (for induction of aggregation during reassembly) (Fig. 19). Figure 19A shows the stability of loaded VLP that had been stored as depicted as analyzed by nDSF. Remarkably, VLP that had been stored in a buffer comprising ammonium sulfate were considerably more stable (i. e. show a higher inflection temperature) compared with the other cryoadditives. Interestingly, this effect was independent of whether the VLP had been frozen fast (black columns) or slowly (dotted columns).

In a next step, these samples were used in a luciferase assay (Fig. 19B). Unexpectedly, the luciferase activity was strongly increased by VLP which were stored in a composition comprising ammonium sulfate. Betaine was also advantageous compared with the sample without cryoadditive and the other tested cryoadditives. Therefore, the use of cryoadditives, especially ammonium sulfate, considerably increased the stability of VLP and increased the infectivity and cargo-delivering efficacy of the loaded VLP. Again, this effect was independent of whether the VLP had been frozen fast (black columns) or slowly (white columns).

### EMBODIMENTS

1. VLP associated with a lysosomal enzyme or an expression vector encoding a lysosomal enzyme for use in a method for the treatment of a lysosomal storage disease in a subject.
2. The VLP for use according to embodiment 1, wherein the VLP does not comprise viral genetic material and the expression vector does not encode viral proteins.
3. The VLP for use according to embodiment 1 or 2, wherein the subject is an animal or a human being, preferably a human being.
4. The VLP for use according to any of the preceding embodiments, wherein the subject has a neurologic deficit.
5. The VLP for use according to any of the preceding embodiments, wherein the lysosomal enzyme is a human enzyme.
6. The VLP for use according to any of the preceding embodiments, wherein the lysosomal enzyme is arylsulfatase A.
7. The VLP for use according to any of the preceding embodiments, wherein the lysosomal enzyme comprises an amino acid sequence which is at least 80 %, more preferably at least 90 % identical to the amino acid sequence according to SEQ ID NO: 1 over its entire length, preferably has the amino acid sequence of SEQ ID NO: 1.
8. The VLP for use according to any of the preceding embodiments, wherein the lysosomal storage disease is Metachromatic Leukodystrophy Disease (MLD).
9. The VLP for use according to any of the preceding embodiments, wherein the expression vector has a size of less than 7 kb, preferably less than 6 kb.
10. The VLP for use according to any of the preceding embodiments, wherein the expression vector has a promoter selected from the group comprising CMV and CAG.
11. The VLP for use according to any of the preceding embodiments, wherein the lysosomal enzyme comprises a nucleotide sequence which is at least 70 %, more preferably at least 80 %, more preferably at least 90 % identical to the nucleotide sequence of SEQ ID NO: 2 over its entire length, preferably is the nucleotide sequence of SEQ ID NO: 2.
12. The VLP for use according to any of the preceding embodiments, wherein the VLP is derived from a human polyoma virus, preferably JCV.
13. The VLP for use according to any of the preceding embodiments, wherein the VLP crosses the blood-brain barrier, preferably the physiologically intact blood-brain barrier, to enter the CNS together with the lysosomal enzyme or the expression vector.
14. The VLP according to embodiment 13, wherein the lysosomal enzyme or the expression vector enters astrocytes, oligodendrocytes, microglia or neurons, preferably oligodendrocytes.
15. The VLP for use according to any of the preceding embodiments, wherein the VLP is administered orally or parenterally, preferably intravenously.
16. The VLP for use according to any of the preceding embodiments, wherein a target cell is contacted with an effective amount of the lysosomal enzyme.
17. The VLP for use according to any of the preceding embodiments, wherein the lysosomal enzyme has a therapeutically effective enzyme activity for at least 10 days, preferably for at least 20 days, more preferably for at least 30 days.
18. The VLP for use according to any of the preceding embodiments, wherein the VLP is composed of VP1 proteins of JC virus.
19. The VLP according to embodiment 18, wherein the VP1 protein comprises an amino acid sequence which is at least 80% identical to the amino acid sequence according to SEQ ID NO: 3 over its entire length, preferably at least 90 % identical.
20. Pharmaceutical composition for use in a method for the treatment of a lysosomal storage disease in a subject, wherein the pharmaceutical composition comprises the VLP according to any of embodiments 1 to 19 and a pharmaceutically acceptable carrier, and/or excipient.
21. Expression vector having a coding region encoding a lysosomal enzyme, preferably human arylsulfatase A, a promoter selected from the group comprising CAG and CMV, preferably being CAG, and having a size of less than 7 kb, preferably less than 6 kb.
22. The expression vector according to embodiment 21, wherein the lysosomal enzyme comprises a nucleotide sequence which is at least 70 %, more preferably at least 80 %, more preferably at least 90 % identical to the nucleotide sequence of SEQ ID NO: 2 over its entire length, preferably is the nucleotide sequence of SEQ ID NO: 2.
23. Method of associating a VLP with a lysosomal enzyme, preferably human arylsulfatase A, or an expression vector encoding a lysosomal enzyme, preferably human arylsulfatase A, wherein the method comprises the following steps:
   a) providing a composition comprising VP1 proteins,
   b) exposing the VP1 proteins of the composition of a) to conditions inducing the VP1 to assemble into VLP,
   c) exposing the VLP of the composition of b) to conditions disassembling the VLP into pentamers,
   d) exposing the pentamers of the composition of c) to conditions inducing the pentamers to reassemble into VLP
   e) exposing the VLP of the composition of d) to conditions disassembling the VLP into pentamers,
   f) exposing the pentamers of the composition of e) to the lysosomal enzyme or the expression vector to conditions inducing the pentamers to assemble into a VLP associated with the lysosomal enzyme or the expression vector.
24. VLP obtainable by the method according to embodiment 23.

### SPECIALLY PREFERRED EMBODIMENTS

1. VLP incorporating a lysosomal enzyme or incorporating an expression vector encoding a lysosomal enzyme for use in a method for the treatment of Metachromatic Leukodystrophy Disease (MLD) in a subject, wherein the VLP is derived from JCV.
2. The VLP for use according to embodiment 1, wherein the VLP does not comprise viral genetic material and the expression vector does not encode viral proteins.
3. The VLP for use according to embodiment 1 or 2, wherein the subject is an animal or a human being, preferably a human being.
4. The VLP for use according to any of the preceding embodiments, wherein the subject has a neurologic deficit.
5. The VLP for use according to any of the preceding embodiments, wherein the lysosomal enzyme is a human enzyme.
6. The VLP for use according to any of the preceding embodiments, wherein the lysosomal enzyme is arylsulfatase A.
7. The VLP for use according to any of the preceding embodiments, wherein the lysosomal enzyme comprises an amino acid sequence which is at least 80 %, more preferably at least 90 % identical to the amino acid sequence according to SEQ ID NO: 1 over its entire length, most preferably has the amino acid sequence of SEQ ID NO: 1.
8. The VLP for use according to any of the preceding embodiments, wherein the expression vector has a size of less than 7 kb, preferably less than 6 kb.
9. The VLP for use according to any of the preceding embodiments, wherein the expression vector has a promoter selected from the group comprising CMV and CAG.
10. The VLP for use according to any of the preceding embodiments, wherein the lysosomal enzyme comprises a nucleotide sequence which is at least 70 %, more preferably at least 80 %, more preferably at least 90 % identical to the nucleotide sequence of SEQ ID NO: 2 over its entire length, preferably is the nucleotide sequence of SEQ ID NO: 2.
11. The VLP for use according to any of the preceding embodiments, wherein the VLP crosses the blood-brain barrier, preferably the physiologically intact blood-brain barrier, to enter the CNS together with the lysosomal enzyme or the expression vector.
12. The VLP according to embodiment 11, wherein the lysosomal enzyme or the expression vector enters astrocytes, oligodendrocytes, microglia or neurons, preferably oligodendrocytes.
13. The VLP for use according to any of the preceding embodiments, wherein the VLP is administered orally or parenterally, preferably intravenously.
14. The VLP for use according to any of the preceding embodiments, wherein a target cell is contacted with an effective amount of the lysosomal enzyme.
15. The VLP for use according to any of the preceding embodiments, wherein the lysosomal enzyme has a therapeutically effective enzyme activity for at least 10 days, preferably for at least 20 days, more preferably for at least 30 days.
16. The VLP for use according to any of the preceding embodiments, wherein the VLP is composed of VP1 proteins of JC virus.
17. The VLP according to special embodiment 16, wherein the VP1 protein comprises an amino acid sequence which is at least 80% identical to the amino acid sequence according to SEQ ID NO: 3 over its entire length, preferably at least 90 % identical.
18. Pharmaceutical composition for use in a method for the treatment of a lysosomal storage disease in a subject, wherein the pharmaceutical composition comprises the VLP according to any of embodiments 1 to 17 and a pharmaceutically acceptable carrier, and/or excipient.
19. Expression vector having a coding region encoding a lysosomal enzyme, preferably human arylsulfatase A, a promoter selected from the group comprising CAG and CMV, preferably being CAG, and having a size of less than 7 kb, preferably less than 6 kb.
20. The expression vector according to embodiment 19, wherein the lysosomal enzyme comprises a nucleotide sequence which is at least 70 %, more preferably at least 80 %, more preferably at least 90 % identical to the nucleotide sequence of SEQ ID NO: 2 over its entire length, preferably is the nucleotide sequence of SEQ ID NO: 2.
21. Method of incorporating a lysosomal enzyme, preferably human arylsulfatase A, or incorporating an expression vector encoding a lysosomal enzyme, preferably human arylsulfatase A, into a VLP, wherein the method comprises the following steps:
   a) providing a composition comprising VP1 proteins,
   b) exposing the VP1 proteins of the composition of a) to conditions inducing the VP1 to assemble into VLP,
   c) exposing the VLP of the composition of b) to conditions disassembling the VLP into pentamers,
   d) exposing the pentamers of the composition of c) to conditions inducing the pentamers to reassemble into VLP
   e) exposing the VLP of the composition of d) to conditions disassembling the VLP into pentamers,
   f) exposing the pentamers of the composition of e) to the lysosomal enzyme or the expression vector to conditions inducing the pentamers to assemble into a VLP associated with the lysosomal enzyme or the expression vector.
22. VLP obtainable by the method according to embodiment 21.
23. A drug delivery system obtainable by the method according to embodiment 21.
24. The VLP for use according to any of embodiments 1 to 17 as a drug delivery system.
25. The VLP for use according to embodiment 24, wherein the method of treatment of MLD does not comprise a step of increasing the permeability of a BBB of the subject to be treated.
26. Method of treating MLD with VLP incorporating a lysosomal enzyme or an expression vector encoding a lysosomal enzyme, wherein the VLP is derived from JCV.
27. VLP incorporating a lysosomal enzyme or an expression vector encoding a lysosomal enzyme for use in delivering the lysosomal enzyme or the expression vector encoding the lysosomal enzyme to a target, wherein the target is the CNS.
28. The VLP for use according to embodiment 27, wherein the lysosomal enzyme or the expression vector encoding the lysosomal enzyme is delivered to a target cell in the CNS, in particular an astrocyte, an oligodendrocyte, a neuron and/or microglia.
29. The VLP for use according to embodiment 28, wherein the lysosomal enzyme is transiently expressed in the target cell.
30. VLP incorporating a lysosomal enzyme or an expression vector encoding a lysosomal enzyme for use in enhancing the lysosomal enzyme activity.
31. Use of the VLP according to any of embodiments 1 to 17 for the manufacture of a medicament for the treatment of MLD.
32. Use of the drug delivery system according to embodiment 23 for the manufacture of a medicament for the treatment of MLD.

## Claims

1. VLP incorporating a lysosomal enzyme or incorporating an expression vector encoding a lysosomal enzyme for use in a method for the treatment of Metachromatic Leukodystrophy Disease (MLD) in a subject, wherein the VLP is derived from JCV.

2. The VLP for use according to claim 1, wherein the lysosomal enzyme is arylsulfatase A.

3. The VLP for use according to claim 1 or 2, wherein the lysosomal enzyme comprises an amino acid sequence which is at least 80 %, more preferably at least 90 % identical to the amino acid sequence according to SEQ ID NO: 1 over its entire length, most preferably has the amino acid sequence of SEQ ID NO: 1.

4. The VLP for use according to any of the preceding claims, wherein the expression vector has a size of less than 7 kb, preferably less than 6 kb.

5. The VLP for use according to any of the preceding claims, wherein the VLP crosses the blood-brain barrier, preferably the physiologically intact blood-brain barrier, to enter the CNS together with the lysosomal enzyme or the expression vector.

6. The VLP for use according to any of the preceding claims, wherein the VLP is composed of VP1 proteins of JC virus.

7. Method of incorporating a lysosomal enzyme, preferably human arylsulfatase A, or incorporating an expression vector encoding a lysosomal enzyme, preferably human arylsulfatase A, into a VLP, wherein the method comprises the following steps:
g) providing a composition comprising VP1 proteins,
h) exposing the VP1 proteins of the composition of a) to conditions inducing the VP1 to assemble into VLP,
i) exposing the VLP of the composition of b) to conditions disassembling the VLP into pentamers,
j) exposing the pentamers of the composition of c) to conditions inducing the pentamers to reassemble into VLP
k) exposing the VLP of the composition of d) to conditions disassembling the VLP into pentamers,
l) exposing the pentamers of the composition of e) to the lysosomal enzyme or the expression vector to conditions inducing the pentamers to assemble into a VLP associated with the lysosomal enzyme or the expression vector.

8. VLP obtainable by the method according to claim 7.

9. A drug delivery system obtainable by the method according to claim 7.

10. The VLP for use according to any of the preceding claims, wherein the method of treatment of MLD does not comprise a step of increasing the permeability of a BBB of the subject to be treated.

11. Method of treating MLD with VLP incorporating a lysosomal enzyme or an expression vector encoding a lysosomal enzyme, wherein the VLP is derived from JCV.


SEQUENCE LISTING

<110>  NEUWAY Pharma GmbH

<120>  VLP for the treatment of a lysosomal storage disease

<130>  61 111 K

<150>  PCT/EP2018/085699
<151>  2018-12-18

<160>  7

<170>  PatentIn version 3.5

<210>  1
<211>  509
<212>  PRT
<213>  Homo sapiens

<400>  1

<210>  2
<211>  1530
<212>  DNA
<213>  Homo sapiens

<400>  2
atgtccatgg gggcaccgcg gtccctcctc ctggccctgg ctgctggcct ggccgttgcc      60

cgtccgccca acatcgtgct gatctttgcc gacgacctcg gctatgggga cctgggctgc     120

tatgggcacc ccagctctac cactcccaac ctggaccagc tggcggcggg agggctgcgg     180

ttcacagact tctacgtgcc tgtgtctctg tgcacaccct ctagggccgc cctcctgacc     240

ggccggctcc cggttcggat gggcatgtac cctggcgtcc tggtgcccag ctcccggggg     300

ggcctgcccc tggaggaggt gaccgtggcc gaagtcctgg ctgcccgagg ctacctcaca     360

ggaatggccg gcaagtggca ccttggggtg gggcctgagg gggccttcct gcccccccat     420

cagggcttcc atcgatttct aggcatcccg tactcccacg accagggccc ctgccagaac     480

ctgacctgct tcccgccggc cactccttgc gacggtggct gtgaccaggg cctggtcccc     540

atcccactgt tggccaacct gtccgtggag gcgcagcccc cctggctgcc cggactagag     600

gcccgctaca tggctttcgc ccatgacctc atggccgacg cccagcgcca ggatcgcccc     660

ttcttcctgt actatgcctc tcaccacacc cactaccctc agttcagtgg gcagagcttt     720

gcagagcgtt caggccgcgg gccatttggg gactccctga tggagctgga tgcagctgtg     780

gggaccctga tgacagccat aggggacctg gggctgcttg aagagacgct ggtcatcttc     840

actgcagaca atggacctga gaccatgcgt atgtcccgag gcggctgctc cggtctcttg     900

cggtgtggaa agggaacgac ctacgagggc ggtgtccgag agcctgcctt ggccttctgg     960

ccaggtcata tcgctcccgg cgtgacccac gagctggcca gctccctgga cctgctgcct    1020

accctggcag ccctggctgg ggccccactg cccaatgtca ccttggatgg ctttgacctc 1080

agccccctgc tgctgggcac aggcaagagc cctcggcagt ctctcttctt ctacccgtcc 1140

tacccagacg aggtccgtgg ggtttttgct gtgcggactg gaaagtacaa ggctcacttc 1200

ttcacccagg gctctgccca cagtgatacc actgcagacc ctgcctgcca cgcctccagc 1260

tctctgactg ctcatgagcc cccgctgctc tatgacctgt ccaaggaccc tggtgagaac 1320

tacaacctgc tggggggtgt ggccggggcc accccagagg tgctgcaagc cctgaaacag 1380

cttcagctgc tcaaggccca gttagacgca gctgtgacct tcggccccag ccaggtggcc 1440

cggggcgagg accccgccct gcagatctgc tgtcatcctg gctgcacccc ccgcccagct 1500

tgctgccatt gcccagatcc ccatgcctga 1530

<210> 3
<211> 354
<212> PRT
<213> Artificial Sequence

<220>
<223> derived from JC virus

<400> 3

Met Ala Pro Thr Lys Arg Lys Gly Glu Pro Lys Asp Pro Val Gln Val
1               5                   10                  15

Pro Lys Leu Leu Ile Arg Gly Gly Val Glu Val Leu Glu Val Lys Thr
            20                  25                  30

Gly Val Asp Ser Ile Thr Glu Val Glu Cys Phe Leu Thr Pro Glu Met
        35                  40                  45

Gly Asp Pro Asp Glu His Leu Arg Gly Phe Ser Lys Ser Ile Ser Ile
    50                  55                  60

Ser Asp Thr Phe Glu Ser Asp Ser Pro Asn Arg Asp Met Leu Pro Cys
65                  70                  75                  80

Tyr Ser Val Ala Arg Ile Pro Leu Pro Asn Leu Asn Glu Asp Leu Thr
                85                  90                  95

Cys Gly Asn Ile Leu Met Trp Glu Ala Val Thr Leu Lys Thr Glu Val
            100                 105                 110

Ile Gly Val Thr Ser Leu Met Asn Val His Ser Asn Gly Gln Ala Thr
        115                 120                 125

His Asp Asn Gly Ala Gly Lys Pro Val Gln Gly Thr Ser Phe His Phe

130                 135                 140

Phe Ser Val Gly Gly Glu Ala Leu Glu Leu Gln Gly Val Val Phe Asn
145                 150                 155                 160

Tyr Arg Thr Lys Tyr Pro Asp Gly Thr Ile Phe Pro Lys Asn Ala Thr
                165                 170                 175

Val Gln Ser Gln Val Met Asn Thr Glu His Lys Ala Tyr Leu Asp Lys
            180                 185                 190

Asn Lys Ala Tyr Pro Val Glu Cys Trp Val Pro Asp Pro Thr Arg Asn
        195                 200                 205

Glu Asn Thr Arg Tyr Phe Gly Thr Leu Thr Gly Gly Glu Asn Val Pro
    210                 215                 220

Pro Val Leu His Ile Thr Asn Thr Ala Thr Thr Val Leu Leu Asp Glu
225                 230                 235                 240

Phe Gly Val Gly Pro Leu Cys Lys Gly Asp Asn Leu Tyr Leu Ser Ala
                245                 250                 255

Val Asp Val Cys Gly Met Phe Thr Asn Arg Ser Gly Ser Gln Gln Trp
            260                 265                 270

Arg Gly Leu Ser Arg Tyr Phe Lys Val Gln Leu Arg Lys Arg Arg Val
        275                 280                 285

Lys Asn Pro Tyr Pro Ile Ser Phe Leu Leu Thr Asp Leu Ile Asn Arg
    290                 295                 300

Arg Thr Pro Arg Val Asp Gly Gln Pro Met Tyr Gly Met Asp Ala Gln
305                 310                 315                 320

Val Glu Glu Val Arg Val Phe Glu Gly Thr Glu Glu Leu Pro Gly Asp
                325                 330                 335

Pro Asp Met Met Arg Tyr Val Asp Lys Tyr Gly Gln Leu Gln Thr Lys
            340                 345                 350

Met Leu

<210>  4
<211>  354
<212>  PRT
<213>  JC virus

<400>  4

Met Ala Pro Thr Lys Arg Lys Gly Glu Arg Lys Asp Pro Val Gln Val
1               5                   10                  15

Pro Lys Leu Leu Ile Arg Gly Gly Val Glu Val Leu Glu Val Lys Thr
            20                  25                  30

Gly Val Asp Ser Ile Thr Glu Val Glu Cys Phe Leu Thr Pro Glu Met
        35                  40                  45

Gly Asp Pro Asp Glu His Leu Arg Gly Phe Ser Lys Ser Ile Ser Ile
    50                  55                  60

Ser Asp Thr Phe Glu Ser Asp Ser Pro Ser Lys Asp Met Leu Pro Cys
65                  70                  75                  80

Tyr Ser Val Ala Arg Ile Pro Leu Pro Asn Leu Asn Glu Asp Leu Thr
                85                  90                  95

Cys Gly Asn Ile Leu Met Trp Glu Ala Val Thr Leu Lys Thr Glu Val
            100                 105                 110

Ile Gly Val Thr Ser Leu Met Asn Val His Ser Asn Gly Gln Ala Ala
        115                 120                 125

His Asp Asn Gly Ala Gly Lys Pro Val Gln Gly Thr Ser Phe His Phe
    130                 135                 140

Phe Ser Val Gly Gly Glu Ala Leu Glu Leu Gln Gly Val Val Phe Asn
145                 150                 155                 160

Tyr Arg Thr Lys Tyr Pro Asp Gly Thr Ile Phe Pro Lys Asn Ala Thr
                165                 170                 175

Val Gln Ser Gln Val Met Asn Thr Glu His Lys Ala Tyr Leu Asp Lys
            180                 185                 190

Asn Lys Ala Tyr Pro Val Glu Cys Trp Val Pro Asp Pro Thr Arg Asn
        195                 200                 205

Glu Asn Thr Arg Tyr Phe Gly Thr Leu Thr Gly Gly Glu Asn Val Pro
    210                 215                 220

Pro Val Leu His Ile Thr Asn Thr Ala Thr Thr Val Leu Leu Asp Glu
225                 230                 235                 240

Phe Gly Val Gly Pro Leu Cys Lys Gly Asp Asn Leu Tyr Leu Ser Ala
                245                 250                 255

Val Asp Val Cys Gly Met Phe Thr Asn Arg Ser Gly Ser Gln Gln Trp
            260                 265                 270

Arg Gly Leu Ser Arg Tyr Phe Lys Val Gln Leu Arg Lys Arg Arg Val
        275                 280                 285

Lys Asn Pro Tyr Pro Ile Ser Phe Leu Leu Thr Asp Leu Ile Asn Arg
    290                 295                 300

Arg Thr Pro Arg Val Asp Gly Gln Pro Met Tyr Gly Met Asp Ala Gln
305                 310                 315                 320

Val Glu Glu Val Arg Val Phe Glu Gly Thr Glu Glu Leu Pro Gly Asp
                325                 330                 335

Pro Asp Met Met Arg Tyr Val Asp Arg Tyr Gly Gln Leu Gln Thr Lys
            340                 345                 350

Met Leu

<210>  5
<211>  1065
<212>  DNA
<213>  Artificial Sequence

<220>
<223>  derived from JC virus

<400>  5
atggctccca ccaagcgcaa gggcgagccc aaggaccccg tgcaagtgcc caagctgctg      60

atccgtggtg gtgtcgaggt gctggaagtc aagaccggcg tggactccat taccgaggtg     120

gagtgcttcc tcacccccga gatgggtgac cctgacgagc acctgagggg cttctccaag     180

tccatctcca tctccgacac cttcgagtcc gactccccca accgtgacat gctgccctgc     240

tactccgtgg ctcgtatccc cctgcccaac ctgaacgagg acctgacttg cggcaacatc     300

ctgatgtggg aggctgtgac cctcaagacc gaggtcatcg gcgtgacttc cctgatgaac     360

gtgcactcca acggccaggc tacccacgac aacggtgctg gcaagcccgt gcagggaacc     420

tccttccact tcttctccgt gggtggcgag gctctggaac tccagggcgt ggtgttcaac     480

taccgtacca agtaccccga cggcaccatc ttccccaaga acgctactgt gcagtcccaa     540

gtgatgaaca ccgagcacaa ggcttacctg gacaagaaca aggcctaccc cgtggagtgc     600

tgggtgcccg accccacccg taacgagaac acccgttact tcggcaccct gaccggtgga     660

gagaacgtgc cccccgtgct gcacatcacc aacaccgcta ccaccgtgct gctggacgag 720

ttcggtgtcg gtcccctgtg caagggcgac aacctgtacc tgtccgctgt ggacgtgtgc 780

ggcatgttca ccaaccgttc cggttcccag cagtggcgtg gcctgtcccg ctacttcaag 840

gtgcagctgc gcaagcgtcg tgtgaagaac ccctacccta tctccttcct gctgaccgac 900

ctgatcaacc gtcgtacccc tcgtgtggac ggccagccca tgtacggcat ggacgctcag 960

gtggaagagg tccgcgtgtt cgagggcacc gaggaattgc ccggcgaccc cgacatgatg 1020

cgttacgtgg acaagtacgg ccagctccag accaagatgc tgtaa 1065

<210> 6
<211> 1065
<212> DNA
<213> JC virus

<400> 6
atggccccaa caaaaagaaa aggagaaagg aaggaccccg tgcaagttcc aaaacttctt 60

ataagaggag gagtagaagt tctagaagtt aaaactgggg ttgactcaat tacagaggta 120

gaatgctttt taactccaga aatgggtgac ccagatgagc atcttagggg ttttagtaag 180

tcaatatcta tatcagatac atttgaaagt gactccccaa atagggacat gcttccttgt 240

tacagtgtgg ccagaattcc actacccaat ctaaatgagg atctaacctg tggaaatata 300

ctcatgtggg aggctgtgac cttaaaaact gaggttatag gggtgacaag tttgatgaat 360

gtgcactcta atgggcaagc aactcatgac aatggtgcag ggaagccagt gcagggcacc 420

agctttcatt ttttttctgt tgggggggag gctttagaat tacagggggt gctttttaat 480

tacagaacaa agtacccaga tggaacaatt tttccaaaga atgccacagt gcaatctcaa 540

gtcatgaaca cagagcacaa ggcgtaccta gataagaaca aagcatatcc tgttgaatgt 600

tgggttcctg atcccaccag aaatgaaaac acaagatatt ttgggacact aacaggagga 660

gaaaatgttc ctccagttct tcatataaca aacactgcca caacagtgtt gcttgatgaa 720

tttggtgttg ggccactttg caaaggtgac aacttatact tgtcagctgt tgatgtctgt 780

ggcatgttta caaacaggtc tggttcccag cagtggagag gactctccag atattttaag 840

gtgcagctaa ggaaaaggag ggttaaaaac ccctacccaa tttctttcct tcttactgat 900

ttaattaaca gaaggactcc tagagttgat gggcagccta tgtatggcat ggatgctcaa 960

gtagaggagg ttagagtttt tgagggaaca gaggagcttc caggggaccc agacatgatg 1020

agatacgttg acaaatatgg acagttgcag acaaaaatgc tgtaa 1065

<210> 7
<211> 1130
<212> DNA
<213> Artificial Sequence

<220>
<223>  pFAR4 polynucleotide

<400>  7
ggtaccgagc tcttacgcgt gctagcccgg gctcgagatc tgcgatctaa gtaagcttcc      60

gggccccccc gccatggggt atcgataatg atatcgaatt cctgcagcgg ggatccacta     120

gttctagagc ggccgccacc gcggtggaca gctggctgtc gacgaagtac tgaccggtct     180

taggcctaaa aaaaatcctt agctttcgct aaggatctgc agtggggtgg ctaatgggat     240

tcgaacccac gacaactgga tttagaatcc agggctctac caactgagct atagccaccg     300

aattgtgcgt tacaagtatt acacaaagtt ttttatgttg agaatatttt tttgatgggg     360

cgcgacttat ttttgatcgt tcgctcaaag aagcggcgcg tcatgaccaa aatcccttaa     420

cgtgagtttt cgttccactg agcgtcagac cccgtagaaa agatcaaagg atcttcttga     480

gatccttttt ttctgcgcgt aatctgctgc ttgcaaacaa aaaaaccacc gctaccagcg     540

gtggtttgtt tgccggatca agagctacca actctttttc cgaaggtaac tggcttcagc     600

agagcgcaga taccaaatac tgttcttcta gtgtagccgt agttaggcca ccacttcaag     660

aactctgtag caccgcctac atacctcgct ctgctaatcc tgttaccagt ggctgctgcc     720

agtggcgata agtcgtgtct taccgggttg gactcaagac gatagttacc ggataaggcg     780

cagcggtcgg gctgaacggg gggttcgtgc acacagccca gcttggagcg aacgacctac     840

accgaactga gatacctaca gcgtgagcta tgagaaagcg ccacgcttcc cgaagggaga     900

aaggcggaca ggtatccggt aagcggcagg gtcggaacag gagagcgcac gagggagctt     960

ccagggggaa acgcctggta tctttatagt cctgtcgggt ttcgccacct ctgacttgag    1020

cgtcgatttt tgtgatgctc gtcagggggg cggagcctat ggaaaaacgc cagcaacgcg    1080

gcctttttac ggttcctggc cttttgctgg ccttttgctc acatgttctt               1130